# EUROPEAN PATENT APPLICATION

(11) **EP 4 553 085 A1**
(43) Date of publication of application: **14.05.2025**
(21) Application number: 23830430.7
(22) Date of filing: 29.06.2023
(51) Int. Cl.: C07K 16/28, A61K 39/395, A61P 35/00

(54) **ISOLATED ANTIGEN-BINDING PROTEIN AND USE THEREOF**

(30) Priority: 30.06.2022 CN 202210758364
(71) Applicant: Hangzhou Sumgen Biotech Co., Ltd., Hangzhou, Zhejiang 310056 (CN); Sumgen Mab (Beijing) Biotech Co., Ltd., Beijing 100176 (CN)
(72) Inventor: LV, Ming, Hangzhou, Zhejiang 310056 (CN); DING, Xiaoran, Hangzhou, Zhejiang 310056 (CN); MIAO, Shiwei, Hangzhou, Zhejiang 310056 (CN); TAN, Bin, Hangzhou, Zhejiang 310056 (CN); TAO, Jun, Hangzhou, Zhejiang 310056 (CN); CUI, Hang, Hangzhou, Zhejiang 310056 (CN)
(74) Representative: Weickmann & Weickmann PartmbB
(86) International application number: PCT/CN2023/104012
(87) International publication number: WO 2024/002266

(57) **Abstract**

Provided is an isolated antigen-binding protein capable of specifically binding to LILRB4 protein. Also provided are a polypeptide comprising the isolated antigen-binding protein, a fusion protein, a drug molecule, a nucleic acid encoding the isolated antigen-binding protein, a vector comprising the isolated antigen-binding protein, a cell comprising the nucleic acid or the vector, a method for preparing the isolated antigen-binding protein, and use of the isolated antigen-binding protein.

## Description

### TECHNICAL FIELD

The present application relates to the field of biological medicines, and specifically relates to an antigen-binding protein targeting LILRB4 and use thereof.

### BACKGROUND ART

Leukocyte immunoglobulin-like receptor B4 (LILRB4) is a member of leukocyte Ig-like receptors (LILRs), which plays a very important role in the function of the immune system under physiological conditions through expression on various immune cells such as T cells and plasma cells. Under pathological conditions, LILRB4 influences the course of various diseases, such as transformation and infiltration of tumors and leukemias, through a variety of signaling pathways. Differential expression of LILRB4 is present in a variety of immune system disorders, such as kawasaki disease, Systemic Lupus Erythematosus (SLE), and sepsis. Recent studies have shown that LILRB4 also plays a role in psychiatric disorders. Due to its important role in the immune system and differential expression in a variety of diseases, LILRB4 has become a potential therapeutic target for a variety of diseases.

Based on the therapeutic potential of LILRB4, there is still a need to develop a plurality of antibodies capable of specifically binding to LILRB4.

### SUMMARY

In one aspect, the present application provides an isolated antigen-binding protein, including one or more of the following properties: 1) specifically recognizing a leukocyte immunoglobulin-like receptor B4 (LILRB4); 2) binding to LILRB4 protein at a K_{D} value of 2×10⁻⁷M or less; 3) blocking the binding activity of LILRB4/APOE; 4) stimulating the activation of CD8+T cell; 5) inhibiting the migration of human mononuclear leukemia cell THP-1; 6) inhibiting the growth and/or metastasis of tumor cells; and 7) blocking the binding of LILRB4 to Fibronectin.

In some embodiments, the LILRB4 is human LILRB4.

In some embodiments, the isolated antigen-binding protein includes at least one CDR in an antibody heavy chain variable region VH, wherein the VH comprises an amino acid sequence as set forth in any one of SEQ ID NO: 81, SEQ ID NO: 33, and SEQ ID NO: 22.

In some embodiments, the isolated antigen-binding protein includes HCDR3, and the HCDR3 includes an amino acid sequence as set forth in SEQ ID NO: 83 or SEQ ID NO: 29.

In some embodiments, the isolated antigen-binding protein includes HCDR3, and the HCDR3 includes an amino acid sequence as set forth in any one of SEQ ID NO: 3, SEQ ID NO: 20 and SEQ ID NO: 29.

In some embodiments, the isolated antigen-binding protein includes HCDR2, and the HCDR2 includes an amino acid sequence as set forth in SEQ ID NO: 2 or SEQ ID NO: 28.

In some embodiments, the isolated antigen-binding protein includes HCDR1, and the HCDR1 includes an amino acid sequence as set forth in SEQ ID NO: 69 (X₁X₂WMX₃, X₁ is D or S; X₂ is A or Y; X₃ is D or H).

In some embodiments, the isolated antigen-binding protein includes HCDR1, and the HCDR1 includes an amino acid sequence as set forth in SEQ ID NO: 1 or SEQ ID NO: 27.

In some embodiments, the isolated antigen-binding protein includes HCDR1, HCDR2 and HCDR3, and the HCDR1, HCDR2 and HCDR3 include the amino acid sequence selected from the group consisting of:
(1) the HCDR1 includes an amino acid sequence as set forth in SEQ ID NO: 1, the HCDR2 includes an amino acid sequence as set forth in SEQ ID NO: 2, and the HCDR3 includes an amino acid sequence as set forth in SEQ ID NO: 3;
(2) the HCDR1 includes an amino acid sequence as set forth in SEQ ID NO: 1, the HCDR2 includes an amino acid sequence as set forth in SEQ ID NO: 2, and the HCDR3 includes an amino acid sequence as set forth in SEQ ID NO: 20; and
(3) the HCDR1 includes an amino acid sequence as set forth in SEQ ID NO: 27, the HCDR2 includes an amino acid sequence as set forth in SEQ ID NO: 28, and the HCDR3 includes an amino acid sequence as set forth in SEQ ID NO: 29.

In some embodiments, the isolated antigen-binding protein includes H-FR1, a C terminus of the H-FR1 is directly or indirectly connected to an N terminus of the HCDR1, and the H-FR1 includes an amino acid sequence as set forth in SEQ ID NO: 73.

In some embodiments, the H-FR1 includes an amino acid sequence as set forth in any one of SEQ ID NO: 4, SEQ ID NO: 17, SEQ ID NO: 30 and SEQ ID NO: 41.

In some embodiments, the isolated antigen-binding protein includes H-FR2, the H-FR2 is located between the HCDR1 and the HCDR2, and the H-FR2 includes an amino acid sequence as set forth in SEQ ID NO: 74.

In some embodiments, the H-FR2 includes an amino acid sequence as set forth in any one of SEQ ID NO: 5, SEQ ID NO: 31, SEQ ID NO: 42 and SEQ ID NO: 53.

In some embodiments, the isolated antigen-binding protein includes H-FR3, the H-FR3 is located between the HCDR2 and the HCDR3, and the H-FR3 includes an amino acid sequence as set forth in SEQ ID NO: 75.

In some embodiments, the H-FR3 includes an amino acid sequence as set forth in any one of SEQ ID NO: 6, SEQ ID NO: 18, SEQ ID NO: 21, SEQ ID NO: 32, SEQ ID NO: 43, SEQ ID NO: 54 and SEQ ID NO: 57.

In some embodiments, the isolated antigen-binding protein includes H-FR4, an N terminus of the H-FR4 is directly or indirectly connected to a C terminus of the HCDR3, and the H-FR4 includes an amino acid sequence as set forth in SEQ ID NO: 76.

In some embodiments, the H-FR4 includes an amino acid sequence as set forth in SEQ ID NO: 7 or SEQ ID NO: 44.

In some embodiments, the isolated antigen-binding protein includes H-FR1, H-FR2, H-FR3 and H-FR4, and the H-FR1, H-FR2, H-FR3 and H-FR4 include the amino acid sequence selected from the group consisting of:
(1) the H-FR1 includes an amino acid sequence as set forth in SEQ ID NO: 4, the H-FR2 includes an amino acid sequence as set forth in SEQ ID NO: 5, the H-FR3 includes an amino acid sequence as set forth in SEQ ID NO: 6, and the H-FR4 includes an amino acid sequence as set forth in SEQ ID NO: 7;
(2) the H-FR1 includes an amino acid sequence as set forth in SEQ ID NO: 17, the H-FR2 includes an amino acid sequence as set forth in SEQ ID NO: 5, the H-FR3 includes an amino acid sequence as set forth in SEQ ID NO: 18, and the H-FR4 includes an amino acid sequence as set forth in SEQ ID NO: 7;
(3) the H-FR1 includes an amino acid sequence as set forth in SEQ ID NO: 4, the H-FR2 includes an amino acid sequence as set forth in SEQ ID NO: 5, the H-FR3 includes an amino acid sequence as set forth in SEQ ID NO: 21, and the H-FR4 includes an amino acid sequence as set forth in SEQ ID NO: 7;
(4) the H-FR1 includes an amino acid sequence as set forth in SEQ ID NO: 30, the H-FR2 includes an amino acid sequence as set forth in SEQ ID NO: 31, the H-FR3 includes an amino acid sequence as set forth in SEQ ID NO: 32, and the H-FR4 includes an amino acid sequence as set forth in SEQ ID NO: 7;
(5) the H-FR1 includes an amino acid sequence as set forth in SEQ ID NO: 41, the H-FR2 includes an amino acid sequence as set forth in SEQ ID NO: 42, the H-FR3 includes an amino acid sequence as set forth in SEQ ID NO: 43, and the H-FR4 includes an amino acid sequence as set forth in SEQ ID NO: 44;
(6) the H-FR1 includes an amino acid sequence as set forth in SEQ ID NO: 41, the H-FR2 includes an amino acid sequence as set forth in SEQ ID NO: 53, the H-FR3 includes an amino acid sequence as set forth in SEQ ID NO: 54, and the H-FR4 includes an amino acid sequence as set forth in SEQ ID NO: 44;
(7) the H-FR1 includes an amino acid sequence as set forth in SEQ ID NO: 41, the H-FR2 includes an amino acid sequence as set forth in SEQ ID NO: 53, the H-FR3 includes an amino acid sequence as set forth in SEQ ID NO: 57, and the H-FR4 includes an amino acid sequence as set forth in SEQ ID NO: 44.

In some embodiments, the isolated antigen-binding protein includes VH, and the VH includes an amino acid sequence as set forth in any one of claims SEQ ID NO: 81, SEQ ID NO: 33 and SEQ ID NO: 22.

In some embodiments, the VH of the isolated antigen-binding protein includes an amino acid sequence as set forth in any one of SEQ ID NO: 8, SEQ ID NO: 19, SEQ ID NO: 22, SEQ ID NO: 33, SEQ ID NO: 45, SEQ ID NO: 55 and SEQ ID NO: 58.

In some embodiments, the isolated antigen-binding protein includes an antibody heavy chain constant region which is derived from an IgG heavy chain constant region.

In some embodiments, the antibody heavy chain constant region is derived from a human IgG heavy chain constant region.

In some embodiments, the antibody heavy chain constant region is derived from a human IgG1 heavy chain constant region or a human IgG4 heavy chain constant region.

In some embodiments, the antibody heavy chain constant region includes an amino acid sequence as set forth in SEQ ID NO: 63.

In some embodiments, the isolated antigen-binding protein includes an antibody heavy chain, and the antibody heavy chain includes amino acid sequences as set forth in any one of SEQ ID NO: 46, SEQ ID NO: 56, SEQ ID NO: 59, SEQ ID NO: 86, SEQ ID NO: 88, SEQ ID NO: 89 and SEQ ID NO: 91.

In some embodiments, the isolated antigen-binding protein includes at least one CDR in an antibody light chain variable region VL, and the VL includes an amino acid sequence as set forth in SEQ ID NO: 82 or SEQ ID NO: 40.

In some embodiments, the isolated antigen-binding protein includes LCDR3, and the LCDR3 includes an amino acid sequence as set forth in SEQ ID NO: 72 (QX₁X₂X₃X₄X₅PX₆T, X₁ is H or Q; X₂ is G or S; X₃ is D, N or W; X₄ is E or T; X₅ is I or L; and X₆ is P or R).

In some embodiments, the LCDR3 includes an amino acid sequence as set forth in any one of SEQ ID NO: 11, SEQ ID NO: 24 or SEQ ID NO: 36.

In some embodiments, the isolated antigen-binding protein includes LCDR2, the LCDR2 includes an amino acid sequence as set forth in SEQ ID NO: 71.

In some embodiments, the LCDR2 includes an amino acid sequence as set forth in SEQ ID NO: 10 or SEQ ID NO: 35.

In some embodiments, the isolated antigen-binding protein includes LCDR1, and the LCDR1 includes an amino acid sequence as set forth in SEQ ID NO: 70 or SEQ ID NO: 34.

In some embodiments, the LCDR1 includes an amino acid sequence as set forth in any one of SEQ ID NO: 9, SEQ ID NO: 23 or SEQ ID NO: 34.

In some embodiments, the isolated antigen-binding protein includes LCDR1, LCDR2 and LCDR3, and the LCDR1, LCDR2 and LCDR3 include the amino acid sequence selected from the group consisting of:
(1) the LCDR1 includes an amino acid sequence as set forth in SEQ ID NO: 9, the LCDR2 includes an amino acid sequence as set forth in SEQ ID NO: 10, and the LCDR3 includes an amino acid sequence as set forth in SEQ ID NO: 11;
(2) the LCDR1 includes an amino acid sequence as set forth in SEQ ID NO: 23, the LCDR2 includes an amino acid sequence as set forth in SEQ ID NO: 10, and the LCDR3 includes an amino acid sequence as set forth in SEQ ID NO: 24; and
(3) the LCDR1 includes an amino acid sequence as set forth in SEQ ID NO: 34, the LCDR2 includes an amino acid sequence as set forth in SEQ ID NO: 35, and the LCDR3 includes an amino acid sequence as set forth in SEQ ID NO: 36.

In some embodiments, the isolated antigen-binding protein includes L-FR1, a C terminus of the L-FR1 is directly or indirectly connected to an N terminus of the LCDR1, and the L-FR1 includes an amino acid sequence as set forth in SEQ ID NO: 77.

In some embodiments, the L-FR1 includes an amino acid sequence as set forth in any one of SEQ ID NO: 12, SEQ ID NO: 25, SEQ ID NO: 37 and SEQ ID NO: 47.

In some embodiments, the isolated antigen-binding protein includes L-FR2, the L-FR2 is located between the LCDR1 and the LCDR2, and the L-FR2 includes an amino acid sequence as set forth in SEQ ID NO: 78.

In some embodiments, the L-FR2 includes an amino acid sequence as set forth in any one of SEQ ID NO: 13, SEQ ID NO: 38 or SEQ ID NO: 48.

In some embodiments, the isolated antigen-binding protein includes L-FR3, the L-FR3 is located between the LCDR2 and the LCDR3, and the L-FR3 includes an amino acid sequence as set forth in SEQ ID NO: 79.

In some embodiments, the L-FR3 includes an amino acid sequence as set forth in any one of SEQ ID NO: 14, SEQ ID NO: 39, SEQ ID NO: 49 and SEQ ID NO: 60.

In some embodiments, the isolated antigen-binding protein includes L-FR4, an N terminus of the L-FR4 is directly or indirectly connected to a C terminus of the LCDR3, and the L-FR4 includes an amino acid sequence as set forth in SEQ ID NO: 80.

In some embodiments, the L-FR4 includes an amino acid sequence as set forth in SEQ ID NO: 15 or SEQ ID NO: 50.

In some embodiments, the isolated antigen-binding protein includes L-FR1, L-FR2, L-FR3 and L-FR4, and the L-FR1, L-FR2, L-FR3 and L-FR4 include the amino acid sequence selected from the group consisting of:
(1) the L-FR1 includes an amino acid sequence as set forth in SEQ ID NO: 12, the L-FR2 includes an amino acid sequence as set forth in SEQ ID NO: 13, the L-FR3 includes an amino acid sequence as set forth in SEQ ID NO: 14, and the L-FR4 includes an amino acid sequence as set forth in SEQ ID NO: 15;
(2) the L-FR1 includes an amino acid sequence as set forth in SEQ ID NO: 25, the L-FR2 includes an amino acid sequence as set forth in SEQ ID NO: 13, the L-FR3 includes an amino acid sequence as set forth in SEQ ID NO: 14, and the L-FR4 includes an amino acid sequence as set forth in SEQ ID NO: 15;
(3) the L-FR1 includes an amino acid sequence as set forth in SEQ ID NO: 37, the L-FR2 includes an amino acid sequence as set forth in SEQ ID NO: 38, the L-FR3 includes an amino acid sequence as set forth in SEQ ID NO: 39, and the L-FR4 includes an amino acid sequence as set forth in SEQ ID NO: 15;
(4) the L-FR1 includes an amino acid sequence as set forth in SEQ ID NO: 47, the L-FR2 includes an amino acid sequence as set forth in SEQ ID NO: 48, the L-FR3 includes an amino acid sequence as set forth in SEQ ID NO: 49, and the L-FR4 includes an amino acid sequence as set forth in SEQ ID NO: 50; and
(5) the L-FR1 includes an amino acid sequence as set forth in SEQ ID NO: 47, the L-FR2 includes an amino acid sequence as set forth in SEQ ID NO: 48, the L-FR3 includes an amino acid sequence as set forth in SEQ ID NO: 60, and the L-FR4 includes an amino acid sequence as set forth in SEQ ID NO: 50.

In some embodiments, the isolated antigen-binding protein includes VL, and the VL includes an amino acid sequence as set forth in SEQ ID NO: 82 or SEQ ID NO: 40.

In some embodiments, the VL includes an amino acid sequence as set forth in any one of SEQ ID NO: 16, SEQ ID NO: 26, SEQ ID NO: 40, SEQ ID NO: 51 and SEQ ID NO: 61.

In some embodiments, the isolated antigen-binding protein includes an antibody light chain constant region which includes a human Igκ constant region.

In some embodiments, the antibody light chain constant region includes an amino acid sequence as set forth in SEQ ID NO: 64.

In some embodiments, the isolated antigen-binding protein includes an antibody light chain, and the antibody light chain includes an amino acid sequence as set forth in any one of SEQ ID NO: 52, SEQ ID NO: 62, SEQ ID NO: 87, SEQ ID NO: 90 and SEQ ID NO: 92.

In some embodiments, the isolated antigen-binding protein includes HCDR1, HCDR2, HCDR3, LCDR1, LCDR2 and LCDR3, and the HCDR1, HCDR2, HCDR3, LCDR1, LCDR2 and LCDR3 include the amino acid sequence selected from the group consisting of:
(1) the HCDR1 includes an amino acid sequence as set forth in SEQ ID NO: 1, the HCDR2 includes an amino acid sequence as set forth in SEQ ID NO: 2, the HCDR3 includes an amino acid sequence as set forth in SEQ ID NO: 3, the LCDR1 includes an amino acid sequence as set forth in SEQ ID NO: 9, the LCDR2 includes an amino acid sequence as set forth in SEQ ID NO: 10, and the LCDR3 includes an amino acid sequence as set forth in SEQ ID NO: 11;
(2) the HCDR1 includes an amino acid sequence as set forth in SEQ ID NO: 1, the HCDR2 includes an amino acid sequence as set forth in SEQ ID NO: 2, the HCDR3 includes an amino acid sequence as set forth in SEQ ID NO: 20, the LCDR1 includes an amino acid sequence as set forth in SEQ ID NO: 23, the LCDR2 includes an amino acid sequence as set forth in SEQ ID NO: 10, and the LCDR3 includes an amino acid sequence as set forth in SEQ ID NO: 24; and
(3) the HCDR1 includes an amino acid sequence as set forth in SEQ ID NO: 27, the HCDR2 includes an amino acid sequence as set forth in SEQ ID NO: 28, the HCDR3 includes an amino acid sequence as set forth in SEQ ID NO: 29, the LCDR1 includes an amino acid sequence as set forth in SEQ ID NO: 34, the LCDR2 includes an amino acid sequence as set forth in SEQ ID NO: 35, and the LCDR3 includes an amino acid sequence as set forth in SEQ ID NO: 36.

In some embodiments, the isolated antigen-binding protein includes VH and VL, and the VH and VL include the amino acid sequence selected from the group consisting of:
(1) the VH includes an amino acid sequence as set forth in SEQ ID NO: 8, and the VL includes an amino acid sequence as set forth in SEQ ID NO: 16;
(2) the VH includes an amino acid sequence as set forth in SEQ ID NO: 19, and the VL includes an amino acid sequence as set forth in SEQ ID NO: 16;
(3) the VH includes an amino acid sequence as set forth in SEQ ID NO: 22, and the VL includes an amino acid sequence as set forth in SEQ ID NO: 26;
(4) the VH includes an amino acid sequence as set forth in SEQ ID NO: 33, and the VL includes an amino acid sequence as set forth in SEQ ID NO: 40;
(5) the VH includes an amino acid sequence as set forth in SEQ ID NO: 45, and the VL includes an amino acid sequence as set forth in SEQ ID NO: 51;
(6) the VH includes an amino acid sequence as set forth in SEQ ID NO: 55, and the VL includes an amino acid sequence as set forth in SEQ ID NO: 51;
(7) the VH includes an amino acid sequence as set forth in SEQ ID NO: 58, and the VL includes an amino acid sequence as set forth in SEQ ID NO: 51; and
(8) the VH includes an amino acid sequence as set forth in SEQ ID NO: 45, and the VL includes an amino acid sequence as set forth in SEQ ID NO: 61.

In some embodiments, the isolated antigen-binding protein includes the antibody heavy chain and the antibody light chain, and the antibody heavy chain and the antibody light chain include the amino acid sequence selected from the group consisting of:
(1) the antibody heavy chain includes an amino acid sequence as set forth in SEQ ID NO: 86, and the antibody light chain includes an amino acid sequence as set forth in SEQ ID NO: 87;
(2) the antibody heavy chain includes an amino acid sequence as set forth in SEQ ID NO: 88, and the antibody light chain includes an amino acid sequence as set forth in SEQ ID NO: 87;
(3) the antibody heavy chain includes an amino acid sequence as set forth in SEQ ID NO: 89, and the antibody light chain includes an amino acid sequence as set forth in SEQ ID NO: 90;
(4) the antibody heavy chain includes an amino acid sequence as set forth in SEQ ID NO: 91, and the antibody light chain includes an amino acid sequence as set forth in SEQ ID NO: 92;
(5) the antibody heavy chain includes an amino acid sequence as set forth in SEQ ID NO: 46, and the antibody light chain includes an amino acid sequence as set forth in SEQ ID NO: 52;
(6) the antibody heavy chain includes an amino acid sequence as set forth in SEQ ID NO: 56, and the antibody light chain includes an amino acid sequence as set forth in SEQ ID NO: 52;
(7) the antibody heavy chain includes an amino acid sequence as set forth in SEQ ID NO: 59, and the antibody light chain includes an amino acid sequence as set forth in SEQ ID NO: 52; and
(8) the antibody heavy chain includes an amino acid sequence as set forth in SEQ ID NO: 46, and the antibody light chain includes an amino acid sequence as set forth in SEQ ID NO: 62.

In some embodiments, the isolated antigen-binding protein includes an antibody or antigen-binding fragment thereof.

In some embodiments, the antibody is selected from the group consisting of a monoclonal antibody, a single-chain antibody, a chimeric antibody, a multispecific antibody, a humanized antibody and a human antibody.

In some embodiments, the antigen-binding fragment is selected from the group consisting of Fab, Fab', F(ab)₂, Fv, F(ab')₂, scFv, di-scFv and dAb fragments.

In another aspect, the present application provides polypeptide which includes the isolated antigen-binding protein.

In another aspect, the present application provides fusion protein which includes the isolated antigen-binding protein.

In another aspect, the present application provides a drug molecule which includes the isolated antigen-binding protein.

In another aspect, the present application provides one or a plurality of isolated nucleic acid molecules which encode the isolated antigen-binding protein.

In another aspect, the present application provides a vector, including the nucleic acid molecules.

In another aspect, the present application provides a cell, including the nucleic acid molecules or the vector.

In another aspect, the present application provides a pharmaceutical composition, including the isolated antigen-binding protein, the polypeptide, the fusion protein, the drug molecule, the nucleic acid molecules, the vector and/or the cell, and optionally a pharmaceutically acceptable carrier.

In some embodiments, the pharmaceutical composition further includes one or more drugs selected from the following group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

In another aspect, the present application provides a method for preparing the isolated antigen-binding protein, including: culturing the cell by expressing with the isolated antigen-binding protein.

In another aspect, the present application provides use of the isolated antigen-binding protein, the polypeptide, the fusion protein, the drug molecule, the nucleic acid molecules, the vector, the cell, and/or the pharmaceutical composition in preparation of a drug which is used for preventing and/or treating diseases and/or symptoms, and the diseases and/or symptoms include diseases and/or symptoms associated to LILRB4 signaling.

In some embodiments, the diseases and/or symptoms include tumor.

In some embodiments, the tumor includes a solid tumor and/or a blood tumor.

In some embodiments, the drug is used in combination with one or more of the drugs selected from the group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

In some embodiments, the diseases and/or symptoms associated to LILRB4 signaling include immune system diseases and/or malignant blood diseases.

In some embodiments, the immune system disease is selected from one or more of the group consisting of kawasaki disease, Systemic Lupus Erythematosus (SLE), and sepsis.

In some embodiments, the solid tumor is selected from one or more of the group consisting of breast cancer, melanoma, colon cancer, lung cancer, kidney cancer, and pancreatic cancer.

In some embodiments, the malignant blood disease is selected from one or more of the group consisting of myelodysplastic syndrome, myeloproliferative tumor, chronic myelomonocytic leukemia (CMML), chronic myelocytic leukemia, or Acute Myelocytic Leukemia (AML), Acute Promyelocytic Leukemia (APL) or M3 AML, acute myelomonocytic leukemia or M4 AML, acute monocytic leukemia or M5 AML, acute myeloblastic leukemia, and polycythemia vera.

In another aspect, the present application provides use of the isolated antigen-binding protein, the polypeptide, the fusion protein, the drug molecule, the nucleic acid molecules, the vector, the cell, and/or the pharmaceutical composition in preventing and/or treating diseases and/or symptoms, and the diseases and/or symptoms include diseases and/or symptoms associated to LILRB4 signaling.

In some embodiments, the diseases and/or symptoms include tumor.

In some embodiments, the tumor includes a solid tumor and/or a blood tumor.

In some embodiments, the isolated antigen-binding protein, the polypeptide, the fusion protein, the drug molecule, the nucleic acid molecules, the vector, the cell, and/or the pharmaceutical composition are used in combination with one or more of the drugs selected from the group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

In some embodiments, the diseases and/or symptoms associated to LILRB4 signaling include immune system diseases and/or malignant blood diseases.

In some embodiments, the immune system disease is selected from one or more of the group consisting of kawasaki disease, Systemic Lupus Erythematosus (SLE), and sepsis.

In some embodiments, the solid tumor is selected from one or more of the group consisting of breast cancer, melanoma, colon cancer, lung cancer, kidney cancer, and pancreatic cancer.

In some embodiments, the malignant blood disease is selected from one or more of the group consisting of myelodysplastic syndrome, myeloproliferative tumor, chronic myelomonocytic leukemia (CMML), chronic myelocytic leukemia, or Acute Myelocytic Leukemia (AML), Acute Promyelocytic Leukemia (APL) or M3 AML, acute myelomonocytic leukemia or M4 AML, acute monocytic leukemia or M5 AML, acute myeloblastic leukemia, and polycythemia vera.

In another aspect, the present application provides a method for preventing and/or treating diseases and/or symptoms, including: applying effective amount of the isolated antigen-binding protein, the polypeptide, the fusion protein, the drug molecule, the nucleic acid molecules, the vector, the cell, and/or the pharmaceutical composition to a subject in need, and the diseases and/or symptoms include diseases and/or symptoms associated to LILRB4 signaling.

In some embodiments, the diseases and/or symptoms include tumor.

In some embodiments, the tumor includes a solid tumor and/or a blood tumor.

In some embodiments, in the method, the isolated antigen-binding protein, the polypeptide, the fusion protein, the drug molecule, the nucleic acid molecules, the vector, the cell, and/or the pharmaceutical composition are used in combination with one or more of the drugs selected from the group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

In some embodiments, the diseases and/or symptoms associated to LILRB4 signaling include immune system diseases and/or malignant blood diseases.

In some embodiments, the immune system disease is selected from one or more of the group consisting of kawasaki disease, Systemic Lupus Erythematosus (SLE), and sepsis.

In some embodiments, the solid tumor is selected from one or more of the group consisting of breast cancer, melanoma, colon cancer, lung cancer, kidney cancer, and pancreatic cancer.

In some embodiments, the malignant blood disease is selected from one or more of the group consisting of myelodysplastic syndrome, myeloproliferative tumor, chronic myelomonocytic leukemia (CMML), chronic myelocytic leukemia, or Acute Myelocytic Leukemia (AML), Acute Promyelocytic Leukemia (APL) or M3 AML, acute myelomonocytic leukemia or M4 AML, acute monocytic leukemia or M5 AML, acute myeloblastic leukemia, and polycythemia vera.

In another aspect, the present application provides a method for diagnosing diseases and/or symptoms associated to expression of an LILRB4 protein in a subject, including: contacting a sample derived from the subject with the isolated antigen-binding protein, and determining the presence and/or amount of a substance capable of specifically binding to the isolated antigen-binding protein in the sample.

In another aspect, the present application provides a method for detecting LILRB4 in a sample, including administering the isolated antigen-binding protein.

In some embodiments, the method is an in vitro and/or ex vivo method.

In another aspect, the present application provides a detection kit, including the isolated antigen-binding protein, and the detection kit is used for detecting the presence and/or amount of LILRB4 protein in a sample or subject.

Those skilled in the art can easily discern other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. The accompanying drawings are briefly described as follows:
FIG. 1 shows binding determination of the isolated antigen binding protein according to the present application and human LILRB4.
FIG. 2 shows a binding specificity detection result of the isolated antigen binding protein according to the present application and an LILRB family protein.
FIG. 3A and FIG. 3B show activity detection of the isolated antigen binding protein according to the present application for blocking LILRB4/APOE binding.
FIG. 4 shows effect detection of the isolated antigen binding protein according to the present application for activating CD8+T cells.
FIG. 5 shows a migration inhibition effect of the isolated antigen binding protein according to the present application on THP-1 cells.
FIG. 6 shows a trend chart of tumor volume growth in a mouse after drug administration.
FIG. 7 shows a ratio of hCD45+ cells in different tissues and organs after applying the isolated antigen binding protein according to the present application.
FIG. 8 shows binding determination of the isolated antigen binding protein according to the present application and LILRB4.
FIG. 9 shows binding determination of the isolated antigen binding protein according to the present application and THP-1 cells.
FIG. 10 shows a migration inhibition effect of the isolated antigen binding protein according to the present application on THP-1 cells.
FIG. 11 shows activity detection of the isolated antigen binding protein according to the present application for blocking LILRB4/APOE binding.
FIG. 12 shows ADCC activity of the isolated antigen binding protein according to the present application on 293T-hLILRB4 cells.
FIG. 13 shows effect detection of the isolated antigen binding protein according to the present application for activating CD8+T cells.
FIG. 14 shows a trend chart of tumor volume growth in a mouse after drug administration.
FIG. 15 shows a fluorescence chart of tumor volume change in a mouse after drug administration.

### DETAILED DESCRIPTION

The embodiments of the invention of the present application will be described below with specific examples. Those skilled in the art can easily understand other advantages and effects of the invention of the present application from the disclosure of the specification.

### Definition of Terms

The present application is further described as follows: in the present application, unless otherwise specified, scientific and technical terms used herein have the meanings commonly understood by those skilled in the art. In addition, the relevant terms of protein and nucleic acid chemistry, molecular biology, cell and tissue culture, microbiology, and immunology used herein and laboratory steps are widely used terms and routine steps in their respective fields. Moreover, in order to better understand the present application, definitions and explanations of related terms are provided below.

In the present application, the term "isolated" usually refers to artificial collection from its natural state. If an "isolated" substance or component occurs in nature, it may be that the natural environment in which the substance or component is has changed, or that the substance has been isolated from the natural environment, or both. For example, a certain unisolated polynucleotide or polypeptide naturally exists in a living animal, and the same polynucleotide or polypeptide with high purity isolated from the natural state is called "isolated". The term "isolated" does not exclude the presence of artificial or synthetic substances, nor other impure substances that do not affect the activity of the substances.

In the present application, the term "isolated antigen-binding protein" generally refers to a protein that has an antigen-binding ability and is collected from a native state by artificial means. The "isolated antigen-binding protein" may include an antigen-binding moiety and, optionally, a scaffold or framework moiety that allows the antigen-binding moiety to employ a conformation which promotes the antigen-binding moiety to bind to an antigen. The antigen-binding protein may include, for example, an antibody-derived protein scaffold or an alternative protein scaffold or artificial scaffold having a transplanted CDR or CDR derivative. Such scaffolds include, but are not limited to, an antibody-derived scaffold including a mutation introduced, for example, to stabilize a three-dimensional structure of the antigen-binding protein, and a fully synthetic scaffold including, for example, a biocompatible polymer. References include, for example, Korndorfer et al., 2003, Proteins: Structure, Function, and Bioinformatics, 53(1): 121-129 (2003); Roque et al., Biotechnol. Prog. 20: 639-654 (2004). In addition, peptide antibody mimics ("PAMs") as well as antibody mimetics -based scaffolds utilizing fibronectin components can be used as the scaffolds.

In the present application, the term "KD" or "K_{D}" generally refers to an "affinity constant" or an "equilibrium dissociation constant" and refers to a value obtained at equilibrium in titration measurements or by dividing a dissociation rate constant (kd) by a binding rate constant (ka). The binding rate constant (ka), dissociation rate constant (kd) and equilibrium dissociation constant (KD) are used for representing the binding affinity of a binding protein (e.g., the isolated antigen binding protein according to the present application) to an antigen (e.g., the LILRB4 protein). Methods of determining the binding and dissociation rate constants are well known in the art. The use of fluorescence-based techniques provides high sensitivity and the ability of examining samples at equilibrium in physiological buffers. For example, the KD value can be determined by Octet, or by other experimental pathways and instruments such as BIAcore (biomolecule interaction analysis) (e.g., instruments obtainable from BIAcore International AB, a GE Healthcare company, Uppsala, Sweden). In addition, the KD value can be determined using KinExA (Kinetic Exclusion Assay) obtainable from Sapidyne Instruments (Boise, Idaho), or using a Surface Plasma Resonator (SPR).

In the present application, the term "LILRB4 protein" generally refers to a Leukocyte Immunoglobulin-like Receptor B4 (LILRB4). In the present application, the term covers intact LILRB4 and functionally active fragments, variants, homologues, analogs, and derivatives thereof.

In the present application, the term "specific binding" or "specific" generally refers to a measurable and reproducible interaction, such as binding between a target and an antibody, which may determine the presence of a target in the presence of a heterogeneous population of molecules (including biomolecules). For example, an antibody that specifically binds to a target (may be an epitope) is an antibody that binds to the target with greater affinity and avidity, more easily, and/or for a greater duration than it binds to other targets. In one embodiment, the extent to which the antibody binds to an unrelated target is less than about 10% of the extent to which the antibody binds to a target, as measured, for example, by Radioimmunoassay (RIA). For example, in the present application, the isolated antigen binding protein is capable of binding to the LILRB4 protein with a dissociation constant (KD) of <2x10⁻⁷M or less. In some embodiments, specific binding may include, but is not required to exclusively bind.

In the present application, the term "variable domain" generally refers to an amino terminus domain of a heavy or light chain of an antibody. The variable domains of the heavy and light chains may be referred to as "VH" and "VL" respectively (or as "VH" and "VL" respectively). These domains are generally the most variable part of the antibody (relative to other antibodies of the same type), and include an antigen binding site.

In the present application, the term "variable" generally refers to a fact that there is a significant difference in sequence between certain segments of the variable domain between antibodies. The V-domain mediates antigen binding and determines the specificity of a particular antibody for its specific antigen. However, variability is not evenly distributed across the entire variable domain. Instead, it is concentrated in three segments called hypervariable regions (CDRs or HVRs) in the light and heavy chain variable domains. The more highly conserved part of the variable domain is called the frame region (FR). The variable domains of the natural heavy and light chains each contain four FR regions, most of which are in a β-fold configuration, connected by three CDRs, which form a ring connection and, in some cases, a part of the β-fold structure. The CDRs in each strand are held together in close proximity by the FR region, and the CDRs from the other strand together promote the formation of antigen-binding sites for the antibody (see Kabat et al, Sequences of Immunological Interest, Fifth Edition, National Institute of Health, Bethesda, Md. (1991)). The constant domain is not directly involved in the binding of antibodies to antigens, but shows various effector functions, such as antibodies involved in antibody-dependent cytotoxicity.

In the present application, the term "antibody" generally refers to immunoglobulins or fragments or derivatives thereof, and covers any polypeptide that includes antigen-binding sites, whether produced in vitro or in vivo. The term includes, but is not limited to, polyclonal, monoclonal, monospecific, multispecific, non-specific, humanized, single-stranded, chimeric, synthetic, recombinant, hybridized, mutated, and transplanted antibodies. Unless otherwise modified by the term "intact", for example, in "intact antibody", for the purposes of the present application, the term "antibody" also includes antibody fragments, such as Fab, F(ab')₂, Fv, scFv, Fd, dAb, and other antibody fragments that maintain antigen-binding function (e.g., specifically bind to LILRB4). Generally, such fragments should include an antigen-binding domain. The basic 4-chain antibody unit is a heterotetrameric glycoprotein consisting of two identical light (L) chains and two identical heavy (H) chains. IgM antibodies consist of 5 basic heterotetrameric units and another polypeptide called a J chain and has 10 antigen-binding sites, while IgA antibodies consist of 2-5 basic 4-chain units that can be combined with the J chain to polymerize to form a polyvalent combination. In a case of IgG, the 4-chain unit is generally about 150,000 daltons. Each L chain is connected to the H chain by a covalent disulfide bond, while the two H chains are connected to each other by one or more disulfide bonds depending on the H chain isoform. Each H and L chain also has regularly spaced intra-chain disulfide bridging bonds. Each H strand has a variable domain (VH) at an N terminus, followed by three constant domains (CH) for α and γ chains, and four CH domains for µ and ε isoforms. Each L chain has a variable domain (VL) at the N terminus and a constant domain at its other end. The VL corresponds to the VH, and CL corresponds to a first constant domain (CH1) of the heavy chain. Specific amino acid residues are treated to form an interface between the light and heavy chain variable domains. The VH and VL pair together to form a single antigen binding site. The structure and properties of different classes of antibodies refer to, for example, page 71 and Chapter 6 in Basic and Clinical Immunology, 8th Edition, Daniel P. Sties, Abba I. Terr and Tristram G. Parsolw (eds), Appleton & Lange, Norwalk, Conn., 1994. The L chain from any vertebrate species can be divided into one of two distinctly different types, called κ and λ, based on the amino acid sequence of its constant domain. Depending on the sequence of amino acids in the constant domain of the heavy chain (CH), immunoglobulins can be classified into different classes or isoforms. There are five classes of immunoglobulins: IgA, IgD, IgE, IgG, and IgM, with heavy chains named α, δ, ε, γ, and µ, respectively. Based on relatively small differences in CH sequence and function, γ and α classes are further divided into subclasses, for example, humans express the following subclasses: IgG1, IgG2A, IgG2B, IgG3, IgG4, IgA1 and IgK1.

In the present application, the term "CDR" generally refers to a region of a variable domain of an antibody, and its sequence is hypervariable and/or forms a structurally defined loop. Generally, the antibody includes six CDRs; three (HCDR1, HCDR2, HCDR3) in VH, and three (LCDR1, LCDR2, LCDR3) in VL. In natural antibodies, HCDR3 and LCDR3 show most of the diversity of the six CDRs, and in particular HCDR3 is considered to function uniquely in imparting fine specificity to the antibody. Reference includes, for example, Xu et al, Immunity 13:37-45 (2000); Johnson and Wu, in Methods in Molecular Biology 248:1-25 (Lo, ed., Human Press, Totowa, N.J., 2003). In fact, naturally occurring camel antibodies consisting only of heavy chains function normally and stably in the absence of light chains. Reference includes, for example, Hamers-Casterman et al., Nature 363:446-448 (1993); Sheriff et al, Nature Struct. Biol. 3:733-736 (1996).

In the present application, the term "FR" generally refers to a more highly conserved portion of an antibody variable domain, which is referred to as a framework region. Generally, the variable domains of natural heavy and light chains each includes four FR regions, four (H-FR1, H-FR2, H-FR3, and H-FR4) in VH, and four (L-FR1, L-FR2, L-FR3, and L-FR4) in VL. For example, the VL of the isolated antigen-binding protein according to the present application can include framework regions L-FR1, L-FR2, L-FR3, and L-FR4. The VH of the isolated antigen-binding protein of the present application can include framework regions H-FR1, H-FR2, H-FR3, and H-FR4.

In the present application, the term 'antigen-binding fragment' generally refers to one or more fragments having the ability to specifically bind to an antigen (e.g., the LILRB4 protein). In the present application, the antigen-binding fragment can include Fab, Fab ', F(ab)₂, Fv fragment, F(ab')₂, scFv, di-scFv, and/or dAb.

In the present application, the term "monoclonal antibody" or "monoclonal antibody (McAb)" or "monoclonal antibody composition" generally refers to antibody molecule products of a single molecule composition. The monoclonal antibody composition shows single binding specificity and affinity for a particular epitope.

In the present application, the term "single-chain antibody" generally refers to a molecule including an antibody heavy chain variable region and a light chain variable region. For example, the single-chain antibody can be linked by a linker (e.g., a linker peptide) from the antibody heavy chain variable region and the light chain variable region.

In the present application, the term "human antibody" generally refers to antibodies with variable region frameworks and CDR regions derived from human germline immunoglobulin sequences. In addition, if the antibody contains a constant region, it is also derived from the human germline immunoglobulin sequence. The human antibody according to the present application may contain amino acid residues that are not encoded by the human germline immunoglobulin sequences, such as mutations introduced by random or point mutations in vitro or by somatic mutations in vivo. However, the term "human antibody" does not include antibodies that CDR sequences derived from other mammalian species are inserted into the human frame sequences.

In the present application, the term "murine antibody" generally refers to an antibody derived from a mouse germline immunoglobulin sequence in the variable region framework and the CDR region. In addition, if the antibody contains a constant region, it is also derived from the mouse germline immunoglobulin sequence. The murine antibody of this application may contain amino acid residues that are not encoded by the mouse germline immunoglobulin sequence, such as mutations introduced by random or point mutations in vitro or by somatic mutations in vivo. However, the term "murine antibody" does not include antibodies inserted into the mouse frame sequence from CDR sequences obtained from other mammalian species.

In the present application, the term "chimeric antibody" generally refers to an antibody obtained by combining non-human genetic material with human genetic material. Or more generally, chimeric antibodies are antibodies that combine the genetic material of one species with the genetic material of another.

In the present application, the term "multispecific antibody" generally refers to an antibody molecule that can recognize two or more antigens or epitopes at the same time. The multispecific antibody can be obtained in a eukaryotic expression system or in a prokaryotic expression system by a chemical conjugation method, a Hybridization-hybridoma method, a genetic engineering antibody preparation method and the like.

In the present application, the term "humanized antibody" generally refers to an antibody derived from a non-human species whose protein sequence has been modified to increase its similarity to a naturally occurring human antibody.

In the present application, the term "fully human antibody" generally refers to a fully humanoral antibody, i.e., both constant and variable regions of the antibody are derived from humans. The fully human antibody can be realized by phage antibody library technology, transgenic mouse prepared human antibody, ribosome display technology, EBV transfecting B cell cloning technology, single B cell cloning and other technologies.

In the present application, the term "direct connection" is opposed to the term "indirect connection", and the term "direct connection" generally refers to direct linking. For example, the direct connection may be situations that substances are directly connected without spacers. The spacer may be a linker. For example, the linker may be a peptide linker. The term "indirect connection" usually refers to situations that substances are not directly connected to each other. For example, the indirect connection may be situations that the materials are linked through the spacers. For example, in the isolated antigen-binding protein according to the present application, the C terminus of the L-FR1 is directly or indirectly connected to the N terminus of the LCDR1.

In the present application, the terms "polypeptide", "peptide" and "protein" are used interchangeably and generally refer to polymers of amino acid residues. The terms may refer to amino acid polymers in which one or more amino acid residues are synthetic chemical mimics of their corresponding natural amino acids, as well as to natural amino acid polymers, those containing modified residues, and unnatural amino acid polymers.

In the present application, the term "fusion protein" generally refers to a protein consisting of two or more polypeptides that, although they are not normally bound in native state, they are bound together by peptide bonds at respective amino and carboxyl termini to form a continuous polypeptide. It is to be understood that these two or more peptide components bind directly or indirectly through peptide linkers/spacers.

In the present application, the term "drug molecule" generally refers to a molecule having desired biological potency. The drug can be prophylactic or curative. The drug molecule may include but is not limited to: protein molecules, including but not limited to peptides, polypeptides, proteins, including post-translationally modified proteins, fusion proteins, antibodies, etc.; small molecules, including inorganic or organic compounds; nucleic acid molecules, including but not limited to double-stranded or single-stranded DNA, or double- or single-stranded RNA (e.g., antisense (molecule), and RNAi), intron sequences, triple helix nucleic acid molecules, and aptamers; or vaccines.

In the present application, the term "isolated nucleic acid molecule" generally refers to nucleotides, deoxyribonucleotides or ribonucleotides in an isolated form of any length, or analogues isolated or artificially synthesized from their native environment.

In the present application, the term "vector" generally refers to a nucleic acid carrying tool into which polynucleotide encoding a certain protein can be inserted and the protein is expressed. The vector can be expressed by transforming, transducing, or transfecting host cells to express the carried elements of genetic material in the host cells. For example, vectors include: plasmids; phagemids; cosmids; artificial chromosomes such as yeast artificial chromosomes (YACs), bacterial artificial chromosomes (BACs) or P1-derived artificial chromosomes (PACs); bacteriophages such as λ phage or M13 phage and animal viruses. The types of animal viruses used as vectors include retroviruses (including lentiviruses), adenoviruses, adeno-associated viruses, herpes viruses (such as herpes simplex virus), poxviruses, baculoviruses, papillomaviruses, and papovaviruses (such as SV40). A vector may contain a variety of elements that control expression, including promoter sequences, transcription initiation sequences, enhancer sequences, selection elements, and reporter genes. In addition, the vector may also contain a replication initiation site. The vector may also include components that facilitate to enter cells, such as viral particles, liposomes, or protein coats, but not just these.

In the present application, the term "cell" generally refers to a single cell, cell line, or cell culture that may be, or has been a recipient of a subject plasmid or vector, which includes a nucleic acid molecule according to the present application or the vector according to the present application. The cell can include the progeny of a single cell. Due to natural, accidental, or intentional mutations, the progeny may not necessarily be exactly the same as the original parent cell (in the morphology of the total DNA complement or in the genome). The cell may include a cell transfected ex vivo with the vector according to the present application. The cell can be a bacterial cell (e.g., E. coli), yeast cells, or other eukaryotic cells, such as COS cells, Chinese hamster ovary (CHO) cells, CHO-K1 cells, LNCAP cells, HeLa cells, HEK293 cells, COS-1 cells, NS0 cells, human non-small cell lung cancer A549 cells, human cutaneous squamous cell carcinoma A431 cells, renal clear cell adenocarcinoma 786-O cells, human pancreatic cancer MIA PaCa-2 cells, Erythroleukemia K562 cells, acute T-cell leukemia Jurkat cells, human breast cancer MCF-7 cells, human breast cancer MDA-MB-231 cells, human breast cancer MDA-MB-468 cells, human breast cancer SKBR3 cells, human ovarian cancer SKOV3 cells, lymphoma U-937 cells, lymphoma Raji cells, human myeloma U266 cells, or human multiple myeloma RPMI8226 cells. In some embodiments, the cells are mammalian cells.

In the present application, the term "pharmaceutical composition" generally refers to compositions that are suitable for administration to sufferers, preferably to human. For example, the pharmaceutical composition according to the present application may include the isolated antigen-binding protein according to the present application, the immunoconjugate according to the present application, the nucleic acid molecule according to the present application, the vector according to the present application and/or the cell according to the present application, and optionally a pharmaceutically acceptable adjuvant. In addition, the pharmaceutical composition may contain suitable preparations of one or more (pharmaceutically effective) carriers, stabilizers, excipients, diluents, solubilizers, surfactants, emulsifiers and/or preservatives. The acceptable components of the composition are preferably non-toxic to the recipient at the doses and concentrations used. The pharmaceutical composition according to the present application includes, but is not limited to, liquid, frozen and lyophilized composition.

In the present application, the term "pharmaceutically acceptable carrier" generally refers to any and all solvents, dispersion media, coatings, isotonic agents, and absorption retardants that are compatible with drug administration and are generally safe, non-toxic, and neither biologically nor otherwise undesirable.

In the present application, the term "subject" generally refers to human or non-human animals, including, but not limited to, cats, dogs, horses, pigs, cows, sheep, rabbits, mice, rats, or monkeys.

In the present application, the term "contain" generally refers to the inclusion of clearly designated features, but not to the exclusion of other elements.

In the present application, the term "approximately" generally refers to a change in the range of 0.5% to 10% above or below a specified value, for example, a change in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### DETAILED DESCRIPTION OF THE INVENTION

### Isolated antigen-binding protein

CDR of an antibody, also known as a complementarity-determining region, is part of a variable region. Amino acid residues in this region can be in contact with antigens or epitopes. The antibody CDR can be determined by a variety of encoding systems, such as CCG, Kabat, Chothia, IMGT, AbM, Kabat/Chothia integrated into account. These encoding systems are known in the art, and it specifically refers to, for example, http://www.bioinf.org.uk/abs/index.html#kabatnum. Those skilled in the art can use different encoding systems to determine the CDR region according to the sequence and structure of the antibody. Using different encoding systems, the CDR regions may differ. In the present application, the CDR encompasses CDR sequences divided according to any CDR division method; the CDR also encompasses variants thereof including amino acid sequences of the CDR after substitution, deletion and/or addition of one or more amino acids, for example, 1-30, 1-20 or 1-10, for another example, 1, 2, 3, 4, 5, 6, 7, 8 or 9 amino acid in substitution, deletion and/or insertion; it also covers congeners, which may be amino acid sequences that have at least about 85% (e.g., at least about 85%, 90%, 91%, 92%, 93%, 94%, 95%, 96%, 97%, 98%, 99% or more) sequence homology to the amino acid sequence of the CDR. For example, the CDR of the isolated antigen-binding protein described in the sequence list of present application can be determined with Kabat.

In one aspect, the present application provides an isolated antigen-binding protein, which may include at least one CDR in VH as set forth in any one of SEQ ID NO: 81, SEQ ID NO: 33 and SEQ ID NO: 22; and/or at least one CDR in VL as set forth in SEQ ID NO: 82 or SEQ ID NO: 40.

X₁VQLX₂QX₃GAEX₄X₅KPGASVKX₆SCKASGYTFTSYWMHWVX₇QX₈PGQGLEWX₉G EINPSNGRTNYNEKFKSX₁₀X₁₁TX₁₂TX₁₃DX₁₄SX₁₅STX₁₆YX₁₇X₁₈LX₁₉SLX₂₀SEDX₂₁AVYYC ARDRYDEGNAMDYWGQGTX₂₂VTVSS (SEQ ID NO: 81). In which, X₁ can be E or Q; X₂ can be Q or V; X₃ can be P or S; X₄ can be L or V; X₅ can be K or V; X₆ can be L or V; X₇ can be K or R; X₈ can be A or R; X₉ can be I or M; X₁₀ can be K or R; X₁₁ can be A or V; X₁₂ can be L or M; X₁₃ can be R or V; X₁₄ can be K or T; X₁₅ can be S or T; X₁₆ can be A or V; X₁₇ can be M or V; X₁₈ can be E or Q; X₁₉ can be N or S; X₂₀ can be R or T; X₂₁ can be S or T; and X₂₂ can be S or T.

DIX₁MTX₂X₃X₄SSLSASX₅GDRVTIX₆CRASQDIX₇NYLNWYQQKPX₈X₉X₁₀X₁₁KLLIYY TSRLHSGVPSRFSGSGSGTX₁₂YX₁₃X₁₄TISX₁₅LX₁₆X₁₇X₁₈DX₁₉ATYX₂₀CQQGX₂₁TLPX₂₂TF GX₂₃GTX₂₄LEIK (SEQ ID NO: 82). In which, X₁ can be Q or V; X₂ can be H or Q; X₃ can be S or T; X₄ can be P or T; X₅ can be L or V; X₆ can be S or T; X₇ can be S or T; X₈ can be D or G; X₉ can be G or K; X₁₀ can be A or T; X₁₁ can be P or V; X₁₂ can be D or E; X₁₃ can be S or T; X₁₄ can be F or L; X₁₅ can be N or S; X₁₆ can be E or Q; X₁₇ can be P or Q; X₁₈ can be D or E; X₁₉ can be F or I; X₂₀can be F or Y; X₂₁ can be D or N; X₂₂ can be P or R; X₂₃ can be G or Q; and X₂₄ can be K or R.

In the present application, the VH may include an amino acid sequence as set forth in any one of SEQ ID NO: 8, SEQ ID NO: 19, SEQ ID NO: 22, SEQ ID NO: 33, SEQ ID NO: 45, SEQ ID NO: 55 and SEQ ID NO: 58.

In the present application, the VL may include an amino acid sequence as set forth in any one of SEQ ID NO: 16, SEQ ID NO: 26, SEQ ID NO: 40, SEQ ID NO: 51 and SEQ ID NO: 61.

In the present application, the HCDR of the isolated antigen-binding protein may be divided in any form, and the HCDR obtained in any form may fall within the protection scope of the present application as long as the VH and/or VL have the same as the amino acid sequence of VH and/or VL shown in any item in the present application.

For example, the division of VH (SEQ ID NO: 8) and/or VL (SEQ ID NO: 16) using different CDR markers can be summarized in the table below.

| | Chothia | AbM | Kabat | Contact | IMGT |
|---|---|---|---|---|---|
| LCDR1 | L24-34 | L24-34 | L24-34 | L30-36 | L27-32 |
| LCDR2 | L50-56 | L50-56 | L50-56 | L46-55 | L50-51 |
| LCDR3 | L89-97 | L89-97 | L89-97 | L89-96 | L89-97 |
| HCDR1 | H26-32 | H26-35 | H31-35 | H30-35 | H26-33 |
| HCDR2 | H52-57 | H50-59 | H50-66 | H47-59 | H51-58 |
| HCDR3 | H99-109 | H99-109 | H99-109 | H97-108 | H97-109 |

For example, the division of VH (SEQ ID NO: 19) and/or VL (SEQ ID NO: 16) using different CDR markers can be summarized in the table below.

| | Chothia | AbM | Kabat | Contact | IMGT |
|---|---|---|---|---|---|
| LCDR1 | L24-34 | L24-34 | L24-34 | L30-36 | L27-32 |
| LCDR2 | L50-56 | L50-56 | L50-56 | L46-55 | L50-51 |
| LCDR3 | L89-97 | L89-97 | L89-97 | L89-96 | L89-97 |
| HCDR1 | H26-32 | H26-35 | H31-35 | H30-35 | H26-33 |
| HCDR2 | H52-57 | H50-59 | H50-66 | H47-59 | H51-58 |
| HCDR3 | H99-109 | H99-109 | H99-109 | H97-108 | H97-109 |

For example, the division of VH (SEQ ID NO: 22) and/or VL (SEQ ID NO: 26) using different CDR markers can be summarized in the table below.

| | Chothia | AbM | Kabat | Contact | IMGT |
|---|---|---|---|---|---|
| LCDR1 | L24-34 | L24-34 | L24-34 | L30-36 | L27-32 |
| LCDR2 | L50-56 | L50-56 | L50-56 | L46-55 | L50-51 |
| LCDR3 | L89-97 | L89-97 | L89-97 | L89-96 | L89-97 |
| HCDR1 | H26-32 | H26-35 | H31-35 | H30-35 | H26-33 |
| HCDR2 | H52-57 | H50-59 | H50-66 | H47-59 | H51-58 |
| HCDR3 | H99-110 | H99-110 | H99-110 | H97-109 | H97-110 |

For example, the division of VH (SEQ ID NO: 33) and/or VL (SEQ ID NO: 40) using different CDR markers can be summarized in the table below.

| | Chothia | AbM | Kabat | Contact | IMGT |
|---|---|---|---|---|---|
| LCDR1 | L24-38 | L24-38 | L24-38 | L30-40 | L27-36 |
| LCDR2 | L54-60 | L54-60 | L54-60 | L50-59 | L54-55 |
| LCDR3 | L93-101 | L93-101 | L93-101 | L93-100 | L93-101 |
| HCDR1 | H26-32 | H26-35 | H31-35 | H30-35 | H26-33 |
| HCDR2 | H52-59 | H50-61 | H50-68 | H47-61 | H51-60 |
| HCDR3 | H101-108 | H101-108 | H101-108 | H99-107 | H99-108 |

In which, Laa-Lbb can refer to an amino acid sequence that is from the N terminus of the variable region of the antibody light chain and ranges from the site aa to the site bb; and Haa-Hbb can refer to an amino acid sequence that is from the N terminus of the variable region of the antibody heavy chain and ranges from the site aa to the site bb. For example, L24-L34 can refer to an amino acid sequence that is from the N terminus of the variable region of the antibody light chain and ranges from the site 24 to the site 34; and H26-H35 can refer to an amino acid sequence that is from the N terminus of the variable region of the antibody heavy chain and ranges from the site 26 to the site 35.

As one of the cases, in the sequence list of the present application, the CDR sequence and the frame region (FR) sequence of the isolated antigen-binding protein are determined by a Kabat numbering mode.

In the present application, the VH of the isolated antigen-binding protein may include HCDR1, HCDR2 and HCDR3.

In the present application, the HCDR1 may include an amino acid sequence as set forth in SEQ ID NO: 69.

X₁X₂WMX₃ (SEQ ID NO: 69). In which, X₁ can be D or S; X₂ can be A or Y; and X₃ can be D or H.

For example, the HCDR1 may include an amino acid sequence as set forth in SEQ ID NO: 1 or SEQ ID NO: 27.

In the present application, the HCDR2 may include an amino acid sequence as set forth in SEQ ID NO: 2 or SEQ ID NO: 28.

In the present application, the HCDR3 may include an amino acid sequence as set forth in SEQ ID NO: 83 or SEQ ID NO: 29.

[-G][DI][RY]Y[DG][EY][DG][NY]AMDY (SEQ ID NO: 83). In which, X₁ may not exist or may be G; X₂ can be D or I; X₃ can be R or Y; X₄ can be D or G; X₅ can be E or Y; X₆ can be D or G; and X₇ can be N or Y.

In the present application, the HCDR1 of the isolated antigen-binding protein may include an amino acid sequence as set forth in SEQ ID NO: 1 or SEQ ID NO: 27, the HCDR2 may include an amino acid sequence as set forth in SEQ ID NO: 2 or SEQ ID NO: 28, and the HCDR3 may include an amino acid sequence as set forth in any one of SEQ ID NO: 3, SEQ ID NO: 20 and SEQ ID NO: 29.

In the present application, the HCDR1 of the isolated antigen-binding protein may include an amino acid sequence as set forth in SEQ ID NO: 1, the HCDR2 may include an amino acid sequence as set forth in SEQ ID NO: 2, and the HCDR3 may include an amino acid sequence as set forth in SEQ ID NO: 3.

In the present application, the HCDR1 of the isolated antigen-binding protein may include an amino acid sequence as set forth in SEQ ID NO: 1, the HCDR2 may include an amino acid sequence as set forth in SEQ ID NO: 2, and the HCDR3 may include an amino acid sequence as set forth in SEQ ID NO: 20.

In the present application, the HCDR1 of the isolated antigen-binding protein may include an amino acid sequence as set forth in SEQ ID NO: 27, the HCDR2 may include an amino acid sequence as set forth in SEQ ID NO: 28, and the HCDR3 may include an amino acid sequence as set forth in SEQ ID NO: 29.

In the present application, the VL of the isolated antigen-binding protein may include LCDR1, LCDR2 and LCDR3.

In the present application, the LCDR1 may include an amino acid sequence as set forth in SEQ ID NO: 70 or SEQ ID NO: 34.

RASQDIX₁NYLN (SEQ ID NO: 70). In which, X₁ can be S or T.

For example, the LCDR1 may include an amino acid sequence as set forth in any one of SEQ ID NO: 9, SEQ ID NO: 23 and SEQ ID NO: 34.

In the present application, the LCDR2 may include an amino acid sequence as set forth in SEQ ID NO: 71.

YX₁SX₂LX₃S (SEQ ID NO: 71). In which, X₁ can be A or T; X₂ can be N or R; and X₃ can be H or Q.

For example, the LCDR2 may include an amino acid sequence as set forth in SEQ ID NO: 10 or SEQ ID NO: 35.

In the present application, the LCDR3 may include an amino acid sequence as set forth in SEQ ID NO: 72.

QX₁X₂X₃X₄X₅PX₆T (SEQ ID NO: 72). For example, X₁ can be H or Q; X₂ can be G or S; X₃ can be D, N or W; X₄ can be E or T; X₅ can be I or L; and X₆ can be P or R.

For example, the LCDR3 may include an amino acid sequence as set forth in any one of SEQ ID NO: 11, SEQ ID NO: 24 and SEQ ID NO: 36.

In the present application, the LCDR1 of the isolated antigen-binding protein may include an amino acid sequence as set forth in SEQ ID NO: 70 or SEQ ID NO: 34, the LCDR2 may include an amino acid sequence as set forth in SEQ ID NO: 71, and the LCDR3 may include an amino acid sequence as set forth in SEQ ID NO: 72 (QX₁X₂X₃X₄X₅PX₆T, X₁ is H or Q; X₂ is G or S; X₃ is D, N or W; X₄ is E or T; X₅ is I or L; and X₆ is P or R).

In the present application, the LCDR1 of the isolated antigen-binding protein may include an amino acid sequence as set forth in SEQ ID NO: 9, the LCDR2 may include an amino acid sequence as set forth in SEQ ID NO: 10, and the LCDR3 may include an amino acid sequence as set forth in SEQ ID NO: 11.

In the present application, the LCDR1 of the isolated antigen-binding protein may include an amino acid sequence as set forth in SEQ ID NO: 23, the LCDR2 may include an amino acid sequence as set forth in SEQ ID NO: 10, and the LCDR3 may include an amino acid sequence as set forth in SEQ ID NO: 24.

In the present application, the LCDR1 of the isolated antigen-binding protein may include an amino acid sequence as set forth in SEQ ID NO: 34, the LCDR2 may include an amino acid sequence as set forth in SEQ ID NO: 35, and the LCDR3 may include an amino acid sequence as set forth in SEQ ID NO: 36.

In the present application, the isolated antigen-binding protein may include HCDR1, HCDR2, HCDR3, LCDR1, LCDR2 and LCDR3.

In the present application, the HCDR1 of the isolated antigen-binding protein may include an amino acid sequence as set forth in SEQ ID NO: 1 or SEQ ID NO: 27, the HCDR2 may include an amino acid sequence as set forth in SEQ ID NO: 2 or SEQ ID NO: 28, the HCDR3 may include an amino acid sequence as set forth in any one of SEQ ID NO: 3, SEQ ID NO: 20 or SEQ ID NO: 29, the LCDR1 may include an amino acid sequence as set forth in any one of SEQ ID NO: 9, SEQ ID NO: 23 or SEQ ID NO: 34, the LCDR2 may include an amino acid sequence as set forth in SEQ ID NO: 10 or SEQ ID NO: 35, and the LCDR3 may include an amino acid sequence as set forth in any one of SEQ ID NO: 11, SEQ ID NO: 24 or SEQ ID NO: 36.

In the present application, the HCDR1, HCDR2, HCDR3, LCDR1, LCDR2 and LCDR3 of the isolated antigen-binding protein may include an amino acid sequence selected from the following group consisting of:
(1) the HCDR1 may include an amino acid sequence as set forth in SEQ ID NO: 1, the HCDR2 may include an amino acid sequence as set forth in SEQ ID NO: 2, the HCDR3 may include an amino acid sequence as set forth in SEQ ID NO: 3, the LCDR1 may include an amino acid sequence as set forth in SEQ ID NO: 9, the LCDR2 may include an amino acid sequence as set forth in SEQ ID NO: 10, and the LCDR3 may include an amino acid sequence as set forth in SEQ ID NO: 11;
(2) the HCDR1 may include an amino acid sequence as set forth in SEQ ID NO: 1, the HCDR2 may include an amino acid sequence as set forth in SEQ ID NO: 2, the HCDR3 may include an amino acid sequence as set forth in SEQ ID NO: 20, the LCDR1 may include an amino acid sequence as set forth in SEQ ID NO: 23, the LCDR2 may include an amino acid sequence as set forth in SEQ ID NO: 10, and the LCDR3 may include an amino acid sequence as set forth in SEQ ID NO: 24; and
(3) the HCDR1 may include an amino acid sequence as set forth in SEQ ID NO: 27, the HCDR2 may include an amino acid sequence as set forth in SEQ ID NO: 28, the HCDR3 may include an amino acid sequence as set forth in SEQ ID NO: 29, the LCDR1 may include an amino acid sequence as set forth in SEQ ID NO: 34, the LCDR2 may include an amino acid sequence as set forth in SEQ ID NO: 35, and the LCDR3 may include an amino acid sequence as set forth in SEQ ID NO: 36.

In the present application, the VH of the isolated antigen-binding protein may include framework regions H-FR1, H-FR2, H-FR3, and H-FR4.

In the present application, a C terminus of the H-FR1 is directly or indirectly connected to an N terminus of the HCDR1, and the H-FR1 includes an amino acid sequence as set forth in SEQ ID NO: 73.

X₁VX₂LX₃X₄X₅GX₆X₇X₈X₉X₁₀PGX₁₁SX₁₂KX₁₃SCX₁₄ASGX₁₅TFX₁₆ (SEQ ID NO: 73). In which, X₁ can be E or Q; X₂ can be K or Q; X₃ can be E, V or Q; X₄ can be E or Q; X₅ can be P or S; X₆ can be A or G; X₇ can be E or G; X₈ can be L or V; X₉ can be K or V; X₁₀ can be K or Q; X₁₁ can be A or G; X₁₂ can be M or V; X₁₃ can be L or V; X₁₄ can be A or K; X₁₅ can be F or Y; and X₁₆ can be S or T.

For example, the H-FR1 may include an amino acid sequence as set forth in any one of SEQ ID NO: 4, SEQ ID NO: 17, SEQ ID NO: 30 and SEQ ID NO: 41.

In the present application, the H-FR2 may be located between the HCDR1 and the HCDR2, and the H-FR2 may include an amino acid sequence as set forth in SEQ ID NO: 74.

WVX₁₀X₂PX₃X₄GLEWX₅X₆ (SEQ ID NO: 74). In which, X₁ can be K or R; X₂ can be A, R or S; X₃ can be E or G; X₄ can be K or Q; X₅ can be I, M or V; and X₆ can be A or G.

For example, the H-FR2 may include an amino acid sequence as set forth in any one of SEQ ID NO: 5, SEQ ID NO: 31, SEQ ID NO: 42 and SEQ ID NO: 53.

In the present application, the H-FR3 may be located between the HCDR2 and the HCDR3, and the H-FR3 may include an amino acid sequence as set forth in SEQ ID NO: 75.

X₁X₂TX₃X₄X₅DX₆SX₇SX₈X₉YX₁₀X₁₁X₁₂X₁₃SLX₁₄X₁₅EDX₁₆X₁₇X₁₈YYCX₁₉R (SEQ ID NO: 75). In which, X₁ can be K or R; X₂ can be A, For V; X₃ can be I, L or M; X₄ can be I, S or T; X₅ can be R or V; X₆ can be D, K or T; X₇ can be K, S or T; X₈ can be S or T; X₉ can be A or V; X₁₀ can be L, M or V; X₁₁ can be E or Q; X₁₂ can be L or M; X₁₃ can be N or S; X₁₄ can be R or T; X₁₅ can be A or S; X₁₆ can be S or T; X₁₇ can be A or G; X₁₈ can be I or V; and X₁₉ can be A or T.

For example, the H-FR3 may include an amino acid sequence as set forth in any one of SEQ ID NO: 6, SEQ ID NO: 18, SEQ ID NO: 21, SEQ ID NO: 32, SEQ ID NO: 43, SEQ ID NO: 54 and SEQ ID NO: 57.

In the present application, an N terminus of the H-FR4 can be connected to a C terminus of the HCDR3, and the H-FR4 includes an amino acid sequence as set forth in SEQ ID NO: 76.

GQGTX₁VTVSS (SEQ ID NO: 76). In which, X₁ can be S or T.

For example, the H-FR4 may include an amino acid sequence as set forth in SEQ ID NO: 7 or SEQ ID NO: 44.

For example, the H-FR1 of the isolated antigen-binding protein according to the present application may include an amino acid sequence as set forth in SEQ ID NO: 73, the H-FR2 may include an amino acid sequence as set forth in SEQ ID NO: 74, the H-FR3 may include an amino acid sequence as set forth in SEQ ID NO: 75, and the H-FR4 may include an amino acid sequence as set forth in SEQ ID NO: 76.

For example, the H-FR1, H-FR2, H-FR3 and H-FR4 of the isolated antigen-binding protein according to the present application may include an amino acid sequence as set forth in the following group consisting of:
(1) the H-FR1 includes an amino acid sequence as set forth in SEQ ID NO: 4, the H-FR2 includes an amino acid sequence as set forth in SEQ ID NO: 5, the H-FR3 includes an amino acid sequence as set forth in SEQ ID NO: 6, and the H-FR4 includes an amino acid sequence as set forth in SEQ ID NO: 7;
(2) the H-FR1 includes an amino acid sequence as set forth in SEQ ID NO: 17, the H-FR2 includes an amino acid sequence as set forth in SEQ ID NO: 5, the H-FR3 includes an amino acid sequence as set forth in SEQ ID NO: 18, and the H-FR4 includes an amino acid sequence as set forth in SEQ ID NO: 7;
(3) the H-FR1 includes an amino acid sequence as set forth in SEQ ID NO: 4, the H-FR2 includes an amino acid sequence as set forth in SEQ ID NO: 5, the H-FR3 includes an amino acid sequence as set forth in SEQ ID NO: 21, and the H-FR4 includes an amino acid sequence as set forth in SEQ ID NO: 7;
(4) the H-FR1 includes an amino acid sequence as set forth in SEQ ID NO: 30, the H-FR2 includes an amino acid sequence as set forth in SEQ ID NO: 31, the H-FR3 includes an amino acid sequence as set forth in SEQ ID NO: 32, and the H-FR4 includes an amino acid sequence as set forth in SEQ ID NO: 7;
(5) the H-FR1 includes an amino acid sequence as set forth in SEQ ID NO: 41, the H-FR2 includes an amino acid sequence as set forth in SEQ ID NO: 42, the H-FR3 includes an amino acid sequence as set forth in SEQ ID NO: 43, and the H-FR4 includes an amino acid sequence as set forth in SEQ ID NO: 44;
(6) the H-FR1 includes an amino acid sequence as set forth in SEQ ID NO: 41, the H-FR2 includes an amino acid sequence as set forth in SEQ ID NO: 53, the H-FR3 includes an amino acid sequence as set forth in SEQ ID NO: 54, and the H-FR4 includes an amino acid sequence as set forth in SEQ ID NO: 44;
(7) the H-FR1 includes an amino acid sequence as set forth in SEQ ID NO: 41, the H-FR2 includes an amino acid sequence as set forth in SEQ ID NO: 53, the H-FR3 includes an amino acid sequence as set forth in SEQ ID NO: 57, and the H-FR4 includes an amino acid sequence as set forth in SEQ ID NO: 44.

In the present application, the VL of the isolated antigen-binding protein may include framework regions L-FR1, L-FR2, L-FR3, and L-FR4.

In the present application, a C terminus of the L-FR1 is directly or indirectly connected to an N terminus of the LCDR1, and the L-FR1 includes an amino acid sequence as set forth in SEQ ID NO: 77.

DIX₁MTX₂X₃X₄X₅SLX₆X₇SX₈GX₉RX₁₀TIX₁₁C (SEQ ID NO: 77). In which, X₁ can be Q or V; X₂ can be H or Q; X₃ can be S or T; X₄ can be P or T; X₅ can be A or S; X₆ can be A or S; X₇ can be A or V; X₈ can be L or V; X₉ can be D or Q; X₁₀ can be A or V; and X₁₁ can be S or T.

For example, the L-FR1 may include an amino acid sequence as set forth in any one of SEQ ID NO: 12, SEQ ID NO: 25, SEQ ID NO: 37 and SEQ ID NO: 47.

In the present application, the L-FR2 may be located between the LCDR1 and the LCDR2, and the L-FR2 may include an amino acid sequence as set forth in SEQ ID NO: 78.

WYQQKPX₁X₂X₃X₄KLLIX₅ (SEQ ID NO: 78). In which, X₁ can be D or G; X₂ can be G, K or Q; X₃ can be A, P or T; X₄ can be P or V; and X₅ can be K or Y.

For example, the L-FR2 may include an amino acid sequence as set forth in any one of SEQ ID NO: 13, SEQ ID NO: 38 and SEQ ID NO: 48.

In the present application, the L-FR3 may be located between the LCDR2 and the LCDR3, and the L-FR3 may include an amino acid sequence as set forth in SEQ ID NO: 79.

GVPX₁RFSGSGSGTX₂X₃X₄X₅X₆IX₇X₈X₉X₁₀X₁₁X₁₂DX₁₃ATYX₁₄C (SEQ ID NO: 79). In which, X₁ can be A or S; X₂ can be D or E; X₃ can be F or Y; X₄ can be S or T; X₅ can be F or L; X₆ can be Nor T; X₇ can be H or S; X₈ can be N, P or S; X₉ can be L or V; X₁₀ can be E or Q; X₁₁ can be E, P or Q; X₁₂ can be D or E; X₁₃ can be F, I or S; and X₁₄ can be For Y.

For example, the L-FR3 may include an amino acid sequence as set forth in any one of SEQ ID NO: 14, SEQ ID NO: 39, SEQ ID NO: 49 and SEQ ID NO: 60.

In the present application, an N terminus of the L-FR4 can be connected to a C terminus of the HCDR3, and the L-FR4 includes an amino acid sequence as set forth in SEQ ID NO: 80.

FGX₁GTX₂LEIK (SEQ ID NO: 80). In which, X₁ can be G or Q; and X₂ can be K or R.

For example, the L-FR4 may include an amino acid sequence as set forth in SEQ ID NO: 15 or SEQ ID NO: 50.

For example, the L-FR1 of the isolated antigen-binding protein according to the present application may include an amino acid sequence as set forth in SEQ ID NO: 77, the L-FR2 may include an amino acid sequence as set forth in SEQ ID NO: 78, the L-FR3 may include an amino acid sequence as set forth in SEQ ID NO: 79, and the L-FR4 may include an amino acid sequence as set forth in SEQ ID NO: 80.

For example, the H-FR1, H-FR2, H-FR3 and H-FR4 of the isolated antigen-binding protein according to the present application may include an amino acid sequence as set forth in the following group consisting of:
(1) the L-FR1 includes an amino acid sequence as set forth in SEQ ID NO: 12, the L-FR2 includes an amino acid sequence as set forth in SEQ ID NO: 13, the L-FR3 includes an amino acid sequence as set forth in SEQ ID NO: 14, and the L-FR4 includes an amino acid sequence as set forth in SEQ ID NO: 15;
(2) the L-FR1 includes an amino acid sequence as set forth in SEQ ID NO: 25, the L-FR2 includes an amino acid sequence as set forth in SEQ ID NO: 13, the L-FR3 includes an amino acid sequence as set forth in SEQ ID NO: 14, and the L-FR4 includes an amino acid sequence as set forth in SEQ ID NO: 15;
(3) the L-FR1 includes an amino acid sequence as set forth in SEQ ID NO: 37, the L-FR2 includes an amino acid sequence as set forth in SEQ ID NO: 38, the L-FR3 includes an amino acid sequence as set forth in SEQ ID NO: 39, and the L-FR4 includes an amino acid sequence as set forth in SEQ ID NO: 15;
(4) the L-FR1 includes an amino acid sequence as set forth in SEQ ID NO: 47, the L-FR2 includes an amino acid sequence as set forth in SEQ ID NO: 48, the L-FR3 includes an amino acid sequence as set forth in SEQ ID NO: 49, and the L-FR4 includes an amino acid sequence as set forth in SEQ ID NO: 50; and
(5) the L-FR1 includes an amino acid sequence as set forth in SEQ ID NO: 47, the L-FR2 includes an amino acid sequence as set forth in SEQ ID NO: 48, the L-FR3 includes an amino acid sequence as set forth in SEQ ID NO: 60, and the L-FR4 includes an amino acid sequence as set forth in SEQ ID NO: 50.

In the present application, the isolated antigen-binding protein may include HCDR1, HCDR2, HCDR3, LCDR1, LCDR2 and LCDR3.

For example, the HCDR1 of the isolated antigen-binding protein may include an amino acid sequence as set forth in SEQ ID NO: 1, the HCDR2 may include an amino acid sequence as set forth in SEQ ID NO: 2, the HCDR3 may include an amino acid sequence as set forth in SEQ ID NO: 3, the LCDR1 may include an amino acid sequence as set forth in SEQ ID NO: 9, the LCDR2 may include an amino acid sequence as set forth in SEQ ID NO: 10, and the LCDR3 may include an amino acid sequence as set forth in SEQ ID NO: 11.

For example, the HCDR1 of the isolated antigen-binding protein may include an amino acid sequence as set forth in SEQ ID NO: 1, the HCDR2 may include an amino acid sequence as set forth in SEQ ID NO: 2, the HCDR3 may include an amino acid sequence as set forth in SEQ ID NO: 20, the LCDR1 may include an amino acid sequence as set forth in SEQ ID NO: 23, the LCDR2 may include an amino acid sequence as set forth in SEQ ID NO: 10, and the LCDR3 may include an amino acid sequence as set forth in SEQ ID NO: 24.

For example, the HCDR1 of the isolated antigen-binding protein may include an amino acid sequence as set forth in SEQ ID NO: 27, the HCDR2 may include an amino acid sequence as set forth in SEQ ID NO: 28, the HCDR3 may include an amino acid sequence as set forth in SEQ ID NO: 29, the LCDR1 may include an amino acid sequence as set forth in SEQ ID NO: 34, the LCDR2 may include an amino acid sequence as set forth in SEQ ID NO: 35, and the LCDR3 may include an amino acid sequence as set forth in SEQ ID NO: 36.

The isolated antigen-binding protein according to the present application may include an antibody light chain variable region VH and an antibody heavy chain variable region VL.

In the present application, the VH may include an amino acid sequence as set forth in any one of SEQ ID NO: 81, SEQ ID NO: 33, and SEQ ID NO: 22. For example, the VH may include an amino acid sequence as set forth in any one of SEQ ID NO: 8, SEQ ID NO: 19, SEQ ID NO: 22, SEQ ID NO: 33, SEQ ID NO: 45, SEQ ID NO: 55 and SEQ ID NO: 58.

In the present application, the VL may include an amino acid sequence as set forth in any one of SEQ ID NO: 82, or SEQ ID NO: 40. For example, the VL may include an amino acid sequence as set forth in any one of SEQ ID NO: 16, SEQ ID NO: 26, SEQ ID NO: 40, SEQ ID NO: 51 and SEQ ID NO: 61.

For example, the VH of the isolated antigen-binding protein may include an amino acid sequence as set forth in SEQ ID NO: 8, and the VL may include an amino acid sequence as set forth in SEQ ID NO: 16.

For example, the VH of the isolated antigen-binding protein may include an amino acid sequence as set forth in SEQ ID NO: 19, and the VL may include an amino acid sequence as set forth in SEQ ID NO: 16.

For example, the VH of the isolated antigen-binding protein may include an amino acid sequence as set forth in SEQ ID NO: 22, and the VL may include an amino acid sequence as set forth in SEQ ID NO: 26.

For example, the VH of the isolated antigen-binding protein may include an amino acid sequence as set forth in SEQ ID NO: 33, and the VL may include an amino acid sequence as set forth in SEQ ID NO: 40.

For example, the VH of the isolated antigen-binding protein may include an amino acid sequence as set forth in SEQ ID NO: 45, and the VL may include an amino acid sequence as set forth in SEQ ID NO: 51.

For example, the VH of the isolated antigen-binding protein may include an amino acid sequence as set forth in SEQ ID NO: 55, and the VL may include an amino acid sequence as set forth in SEQ ID NO: 51.

For example, the VH of the isolated antigen-binding protein may include an amino acid sequence as set forth in SEQ ID NO: 58, and the VL may include an amino acid sequence as set forth in SEQ ID NO: 51.

For example, the VH of the isolated antigen-binding protein may include an amino acid sequence as set forth in SEQ ID NO: 45, and the VL may include an amino acid sequence as set forth in SEQ ID NO: 61.

In the present application, the isolated antigen-binding protein may include an antibody heavy chain constant region which can be derived from a human IgG heavy chain constant region.

In some embodiments, the isolated antigen-binding protein may include an antibody heavy chain constant region which can be derived from a human IgG1 heavy chain constant region. In another embodiments, the isolated antigen-binding protein may include an antibody heavy chain constant region which can be derived from a human IgG4 heavy chain constant region.

For example, the antibody heavy chain constant region may include an amino acid sequence as set forth in SEQ ID NO: 63.

In the present application, the isolated antigen-binding protein may include an antibody light chain constant region which can be derived from a human Igκ constant region. For example, the antibody light chain constant region may include an amino acid sequence as set forth in SEQ ID NO: 64.

In the present application, the isolated antigen-binding protein may include an antibody heavy chain HC, and the HC may include an amino acid sequence as set forth in any one of SEQ ID NO: 46, SEQ ID NO: 56, SEQ ID NO: 59, SEQ ID NO: 86, SEQ ID NO: 88, SEQ ID NO: 89 and SEQ ID NO: 91.

In the present application, the isolated antigen-binding protein may include an antibody light chain LC, and the LC may include an amino acid sequence as set forth in any one of SEQ ID NO: 52, SEQ ID NO: 62, SEQ ID NO: 87, SEQ ID NO: 90 and SEQ ID NO: 92.

The isolated antigen-binding protein according to the present application may include an antibody heavy chain and an antibody light chain.

For example, the antibody heavy chain may include an amino acid sequence as set forth in SEQ ID NO: 86, and the antibody light chain may include an amino acid sequence as set forth in SEQ ID NO: 87.

For example, the antibody heavy chain may include an amino acid sequence as set forth in SEQ ID NO: 88, and the antibody light chain may include an amino acid sequence as set forth in SEQ ID NO: 87.

For example, the antibody heavy chain may include an amino acid sequence as set forth in SEQ ID NO: 89, and the antibody light chain may include an amino acid sequence as set forth in SEQ ID NO: 90.

For example, the antibody heavy chain may include an amino acid sequence as set forth in SEQ ID NO: 91, and the antibody light chain may include an amino acid sequence as set forth in SEQ ID NO: 92.

For example, the antibody heavy chain may include an amino acid sequence as set forth in SEQ ID NO: 46, and the antibody light chain may include an amino acid sequence as set forth in SEQ ID NO: 52.

For example, the antibody heavy chain may include an amino acid sequence as set forth in SEQ ID NO: 56, and the antibody light chain may include an amino acid sequence as set forth in SEQ ID NO: 52.

For example, the antibody heavy chain may include an amino acid sequence as set forth in SEQ ID NO: 59, and the antibody light chain may include an amino acid sequence as set forth in SEQ ID NO: 52.

For example, the antibody heavy chain may include an amino acid sequence as set forth in SEQ ID NO: 46, and the antibody light chain may include an amino acid sequence as set forth in SEQ ID NO: 62.

In addition, it is to be noted that the isolated antigen-binding protein according to the present application may contain heavy chain and/or light chain sequences with one or more conserved sequence modifications with the antigen-binding protein according to the present application. The term "conserved sequence modification" refers to an amino acid modification that does not significantly affect or change the binding properties of the antibody. Such conserved modifications include amino acid substitutions, additions, and deletions. The modification can be introduced into the isolated antigen-binding proteins according to the present application by standard techniques known in the art, such as point mutations and PCR-mediated mutations. The conserved amino acid substitution refers to the substitution of amino acid residues with amino acid residues having similar side chains. The group of amino acid residues having similar side chains is known in the art. These groups of amino acid residues include those with basic side chains (e.g., lysine, arginine, and histidine), acidic side chains (e.g., aspartic acid, and glutamic acid), side chains without polarity (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine) and aromatic side chains (e.g., tyrosine, phenylalanine, tryptophan, and histidine). In some embodiments, one or more amino acid residues in the CDR region of the isolated antigen-binding protein according to the present application may be substituted with other amino acid residues of the same side chain group. Those skilled in the art know that some conserved sequence modifications do not cause antigen binding to disappear, specifically referring to, for example, Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem. 32:6862-35; Adib-Conquy et al., (1998) Int. Immunol.10:341-6 and Beers et al., (2000) Clin. Can. Res. 6:2835-43.

It is to be understood that the protein, polypeptide and/or amino acid sequence according to the present application contain at least the following ranges: variants or homologs having the same or similar functions as the protein or polypeptide.

In the present application, the variant may be a protein or polypeptide that has been substituted, deleted, or added with one or more amino acids in the amino acid sequence of the protein and/or the polypeptide (e.g., the isolated antigen binding protein according to the present application). For example, the variant may include a protein or polypeptide that has amino acid change caused by substitution, deletion, and/or insertion of at least one, e.g., 1-30, 1-20, or 1-10, yet e.g., 1, 2, 3, 4, or 5 amino acids. The functional variant may substantially maintain the biological properties of the protein or polypeptide prior to change (e.g., substitution, deletion, or addition). For example, the functional variant may maintain at least 60%, 70%, 80%, 90%, or 100% biological activity of the protein or polypeptide prior to change (e.g., the ability to specifically bind to the LILRB4 protein).

In the present application, the homologue may be a protein or polypeptide having at least about 80% (e.g., having at least about 85%, about 90%, about 91%, about 92%, about 93%, about 94%, about 95%, about 96%, about 97%, about 98%, about 99%, or more) sequence homology with the amino acid sequence of the protein and/or the polypeptide (e.g., the antibody or antigen-binding fragment thereof according to the present application).

In the present application, the homology generally refers to the degree of similarity or association between two or more sequences. The "percentage of sequence homology" can be calculated by comparing two sequences to be aligned in a comparison window, determining the number of sites in which the same nucleic acid base (e.g., A, T, C, G, and I) or the same amino acid residue (e.g., Ala, Pro, Ser, Thr, Gly, Val, Leu, Ile, Phe, Tyr, Trp, Lys, Arg, His, Asp, Glu, Asn, Gln, Cys and Met) are present in the two sequences to obtain the number of matching sites, dividing the number of matching sites by the total number of sites in the comparison window (i.e., window size), and multiplying the result by 100 to produce the percentage of sequence homology. Alignment for the purpose of determining percent sequence homology can be accomplished in a variety of ways known in the art, for example, using publicly available computer software such as BLAST, BLAST-2, ALIGN or Megalign (DNASTAR) software. One skilled in the art can determine appropriate parameters for aligning sequences, including any algorithms required to achieve maximal alignment within the full-length sequences being compared or within the sequence region of interest. The homology can also be determined by the following methods: FASTA and BLAST. A description of the FASTA algorithm can be found in W. R. Pearson and D. J. Lipman, "Improved Tool for Biological Sequence Comparison", Proceedings of the National Academy of Sciences (Proc. Natl. Acad. Sci. ), 85: 2444-2448, 1988; and D. J. Lipman and W. R. Pearson, "Fast and Sensitive Protein Similarity Search", Science, 227: 1435-1441, 1989. A description of the BLAST algorithm can be found in S. Altschul, W. Gish, W. Miller, E. W. Myers and D. Lipman, "Basic Local Alignment Search Tool," Journal of Molecular Biology, 215:403-410, 1990.

In the present application, the isolated antigen-binding protein may include an antibody or antigen-binding fragment thereof. For example, the isolated antigen-binding protein according to the present application may include but is not limited to a recombinant antibody, a monoclonal antibody, a human antibody, a murine antibody, a humanized antibody, a chimeric antibody, a single-chain antibody, a multi-antibody, an Fv fragment, an scFv fragment, an Fab fragment, an Fab' fragment, an F(ab')₂ fragment and a camelized single domain antibody.

In the present application, the antibody may be a humanized antibody. In other words, the isolated antigen binding protein according to the present application may be an antibody or a variant, derivative, analog or fragment thereof that immunospecifically binds to a related antigen (e.g., human LILRB4) and includes a Framework (FR) region which substantially has the amino acid sequence of a human antibody and a Complementarity Determining Region (CDR) which substantially has the amino acid sequence of a non-human antibody. In a case of CDR, the "substantially" refers to that the amino acid sequence of the CDR is at least 80%, preferably at least 85%, at least 90%, at least 95%, at least 98% or at least 99% identical to the amino acid sequence of the non-human antibody CDR. The humanized antibody may substantially include all at least one and generally two variable domains (Fab, Fab', F(ab')2, FabC, and Fv), in which all or substantially all of the CDR regions correspond to the CDR region of a non-human immunoglobulin (i.e., antibody) and all or substantially all of the framework regions are framework regions having a consensus sequence for a human immunoglobulin. Preferably, the humanized antibody further includes at least a portion of an immunoglobulin constant region (e.g., Fc), generally a constant region of a human immunoglobulin. In some embodiments, the humanized antibody contains at least variable domains of a light chain and a heavy chain. The antibody may further includes CH1, hinge, CH2, CH3 and CH4 regions of the heavy chain. In some embodiments, the humanized antibody contains only a humanized light chain. In some embodiments, the humanized antibody contains only a humanized heavy chain. In particular embodiments, the humanized antibody contains only humanized variable domains of a light chain and/or a humanized heavy chain.

In the present application, the antigen-binding fragment can include Fab, Fab ', F(ab)₂, Fv fragment, F(ab')₂, scFv, di-scFv, and/or dAb.

In the present application, the isolated antigen-binding protein may have one or more of the following properties: 1) specifically recognizing a leukocyte immunoglobulin-like receptor B4 (LILRB4); 2) binding to LILRB4 protein at a K_{D} value of 2×10⁻⁷M or less; 3) blocking the binding activity of LILRB4/APOE; 4) stimulating the activation of CD8+T cell; 5) inhibiting the migration of human mononuclear leukemia cell THP-1; 6) inhibiting the growth and/or metastasis of tumor cells; and 7) blocking the binding of LILRB4 to Fibronectin.

In the present application, the isolated antigen-binding protein can bind to the LILRB4 protein at a K_{D} value of 2×10⁻⁷M or less. For example, the K_{D} value of the isolated antigen-binding protein according to the present application binding to human LILRB4 protein may be ≤2×10⁻⁷M, ≤1.5×10⁻⁷M, ≤1×10⁻⁷M, ≤9×10⁻⁸M, ≤8×10⁻⁸M, ≤7×10⁻⁸M, ≤6×10⁻⁸M, ≤5×10⁻⁸M, ≤4×10⁻⁸M, ≤3×10⁻⁸M, ≤2×10⁻⁸M, ≤1.5×10⁻⁸M, ≤1.2×10⁻⁸M, ≤1.15×10⁻⁸M, ≤1.1×10⁻⁸M, ≤1.05×10⁻⁸M, ≤1×10⁻⁸M, ≤5×10⁻⁹M or ≤1×10⁻⁹M.

In the present application, the KD value may also be determined by ELISA, competitive ELISA, or BIACORE or KINEXA.

In the present application, the isolated antigen-binding protein is capable of specifically binding to a human LILRB4 protein. The specific binding may be determined by FACS.

In the present application, the LILRB4 protein may be a human LILRB4 protein (GenBank accession number: CAG46845.1).

The LILRB4 protein may include a variant of the LILRB4 protein. For example, the variant may be: 1) a protein or polypeptide that has been substituted, deleted, or added with one or more amino acids in the amino acid sequence of the LILRB4 protein; and 2) a protein or polypeptide that has at least about 85% (e.g., has at least about 85%, about 90%, about 91%, about 92%, about 93%, about 94%, about 95%, about 96%, about 97%, about 98%, about 99%, or greater) sequence homology with the LILRB4 protein.

The LILRB4 protein may also include a functionally active fragment of the LILRB4 protein.

### Polypeptide, fusion protein, drug molecule, nucleic acid molecule, vector, cell and pharmaceutical composition

In another aspect, the present application provides polypeptide which includes the isolated antigen-binding protein.

In another aspect, the present application provides fusion protein which includes the isolated antigen-binding protein.

In another aspect, the present application provides a drug molecule which includes the isolated antigen-binding protein.

In another aspect, the present application provides one or a plurality of isolated nucleic acid molecules which encode the isolated antigen-binding protein. The isolated one nucleic acid molecule or molecules according to the present application may be nucleotides, deoxyribonucleotides or ribonucleotides in isolated form of any length, or analogs isolated from native environment or artificially synthesized, but may encode the isolated antigen-binding protein according to the present application.

In another aspect, the present application provides a vector, including the nucleic acid molecules according to the present application. The vector can be expressed by transforming, transducing, or transfecting host cells to express the carried elements of genetic material in the host cells. For example, vectors include: plasmids; phagemids; cosmids; artificial chromosomes such as yeast artificial chromosomes (YACs), bacterial artificial chromosomes (BACs) or P1-derived artificial chromosomes (PACs); bacteriophages such as λ phage or M13 phage and animal viruses. The types of animal viruses used as vectors include retroviruses (including lentiviruses), adenoviruses, adeno-associated viruses, herpes viruses (such as herpes simplex virus), poxviruses, baculoviruses, papillomaviruses, and papovaviruses (such as SV40). For another example, a vector may contain a variety of elements that control expression, including promoter sequences, transcription initiation sequences, enhancer sequences, selection elements, and reporter genes. In addition, the vector may also contain a replication initiation site. In addition, the vector may also include components that facilitate to enter cells, such as viral particles, liposomes, or protein coats, but not just these.

In another aspect, the present application provides a cell, including the nucleic acid molecules according to the present application or the vector according to the present application. The cell may include the progeny of a single cell. Due to natural, accidental or intentional mutations, the progeny may not necessarily be identical to the original mother cell (in the morphology or genome of the total DNA complement). In some embodiments, the cell may also include cells transfected ex vivo with the vector according to the present application. In some embodiments, the cell may be bacterial cells (e.g., E. Coli), yeast cells or other eukaryotic cells.

In another aspect, the present application provides a pharmaceutical composition, including the isolated antigen-binding protein according to the present application, the polypeptide according to the present application, the fusion protein according to the present application, the nucleic acid molecules according to the present application, the vector according to the present application and/or the cell according to the present application, and optionally a pharmaceutically acceptable carrier.

In some embodiments, the pharmaceutical composition may contain suitable preparations of one or more (pharmaceutically effective) carriers, stabilizers, excipients, diluents, solubilizers, surfactants, emulsifiers and/or preservatives. The acceptable components of the composition are preferably non-toxic to the recipient at the doses and concentrations used. The pharmaceutical composition according to the present application includes, but is not limited to, liquid, frozen and lyophilized composition.

In some embodiments, the pharmaceutically acceptable adjuvant can include any and all solvents, dispersion media, coatings, isotonic agents, and absorption retardants that are compatible with drug administration and are generally safe, non-toxic, and neither biologically nor otherwise undesirable.

In some embodiments, the pharmaceutical composition can be administered parenterally, percutaneously, intracavitary, intraarterially, intrathecally, and/or intranasally, or injected directly into tissue. For example, the pharmaceutical composition can be administered to the patient or subject by infusion or injection. In some embodiments, the administration of the pharmaceutical composition can be performed in various ways, such as intravenous, intraperitoneal, subcutaneous, intramuscular, topical, or intradermal administration. In some embodiments, the pharmaceutical composition can be administered uninterruptedly. The uninterrupted (or continuous) administration can be achieved by a small pump system worn by the patient to measure the flow of a therapeutic agent into the patient, such as WO2015/036583.

In the present application, the pharmaceutical composition further includes one or more drugs selected from the following group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

### Preparation method and use

In another aspect, the present application provides a method for preparing the isolated antigen-binding protein according to the present application, including: culturing the cell according to the present application by expressing with the isolated antigen-binding protein according to the present application.

In another aspect, the present application provides use of the isolated antigen-binding protein, the polypeptide, the fusion protein, the drug molecule, the nucleic acid molecules, the vector, the cell, and/or the pharmaceutical composition in preparation of a drug which is used for preventing and/or treating diseases and/or symptoms.

In another aspect, the present application provides the isolated antigen-binding protein, the polypeptide, the fusion protein, the drug molecule, the nucleic acid molecules, the vector, the cell, and/or the pharmaceutical composition, which are used for preventing and/or treating diseases and/or symptoms.

In another aspect, the present application provides a method for preventing and/or treating diseases and/or symptoms, including: applying effective amount of the isolated antigen-binding protein, the polypeptide, the fusion protein, the drug molecule, the nucleic acid molecules, the vector, the cell, and/or the pharmaceutical composition to a subject in need.

In the present application, the administration can be carried out in different ways, such as intravenous, intratumoral, intraperitoneal, subcutaneous, intramuscular, topical or intradermal administration.

In the present application, the diseases and/or symptoms may include diseases and/or symptoms associated to LILRB4 signaling.

In the present application, the diseases and/or symptoms include tumor.

In the present application, the diseases and/or symptoms include positive LILRB4 diseases and/or symptoms.

In the present application, the tumor includes a solid tumor and/or a blood tumor.

In the present application, the diseases and/or symptoms associated to LILRB4 signaling may include immune system diseases and/or malignant blood diseases.

In the present application, the immune system disease is selected from one or more of the group consisting of kawasaki disease, Systemic Lupus Erythematosus (SLE), and sepsis.

In the present application, the solid tumor is selected from one or more of the group consisting of breast cancer, melanoma, colon cancer, lung cancer, kidney cancer, and pancreatic cancer.

In the present application, the malignant blood disease is selected from one or more of the group consisting of myelodysplastic syndrome, myeloproliferative tumor, chronic myelomonocytic leukemia (CMML), chronic myelocytic leukemia, or Acute Myelocytic Leukemia (AML), Acute Promyelocytic Leukemia (APL) or M3 AML, acute myelomonocytic leukemia or M4 AML, acute monocytic leukemia or M5 AML, acute myeloblastic leukemia, and polycythemia vera.

In the present application, the isolated antigen-binding protein, the polypeptide, the fusion protein, the drug molecule, the nucleic acid molecules, the vector, the cell, the pharmaceutical composition , and/or the medication can be used in combination with one or more drugs selected from the following group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

In another aspect, the present application provides use of the antibody or antigen-binding fragment thereof in preparation of a diagnostic agent for diagnosing diseases or conditions associated with expression of an LILRB4 protein.

In another aspect, the present application provides use of the isolated antigen-binding protein in diagnosing of diseases or conditions associated with expression of an LILRB4 protein.

In the present application, the diagnostic agent can be used alone or in combination with an instrument, appliance, device or system. In the disease prevention, diagnosis, therapeutic monitoring, prognosis observation, health status evaluation and prediction of genetic diseases, the diagnostic agent can be used for in vitro detection of a human sample (e.g., various body fluids, cells, and tissue samples). The diagnostic agent can be selected from the group consisting of a reagent, a kit, a calibration product and a quality control.

The method for in vitro detection can be selected from the group consisting of Western Blot, ELISA and immunohistochemistry. For example, the reagent can include a reagent capable of measuring the expression amount of the LILRB4 protein. For example, the reagent can be selected from the group consisting of: a reagent for performing Western Blot, a reagent for performing ELISA and a reagent for performing immunohistochemistry.

In another aspect, the present application provides a detection kit including the isolated antigen binding protein, and the detection kit is used for detecting the presence and/or amount of LILRB4 protein in a sample. In particular, the present application relates to an immunodetection kit for use in combination with an immunodetection method such as ELISA, immunohistochemistry, western blotting, and flow cytometry by using the antibodies of the present application.

The kit includes the antibody according to the present application and is used for detecting LILRB4-related cancer cells, in particular, in the immunodetection kit, the antibody according to the present application is treated as a first antibody binding to the LILRB4 and optionally an immunodetection reagent, in a suitable container member.

In some embodiments, the antibody may be pre-bound to a solid carrier, such as a column matrix and/or a microtiter plate aperture.

The immunodetection reagents in the kit may be in any of a variety of forms, including those detectable markers that bind or are linked to a given antibody. The kit may further include detectable markers that bind or are linked to a secondary binding ligand. Exemplary secondary ligands are those secondary antibodies that have binding affinity for the first antibody.

Other immunodetection reagents suitable for use in the kit according to the present application include a two-component reagent including a secondary antibody having a binding affinity for the first antibody, and a third antibody having a binding affinity for the second antibody, and the third antibody being linked to the detectable marker. As described above, a variety of exemplary labels are known in the art and all such markers can be used in conjunction with the present application.

The kit can further contain a suitable aliquot of LILRB4 composition, whether marked or unmarked, which can be used for preparing a standard curve for detection analysis. The kit can contain an antibody-marked conjugate in a fully conjugated form and an intermediate form, or a respective portion of which the conjugation is completed by a user of the kit. The components of the kit can be encapsulated in an aqueous medium or in lyophilized form.

The container member of the kit will generally include at least one vial, tube, flask, bottle, syringe, or other container member in which an antibody or preferably a suitable aliquot of antibody can be placed. The kit according to the present application will also generally include a member for containing the antibody, antigen, and any other reagent container, which is in a sealed form for commercial sale. Such containers can include injection molded or blow molded plastic containers in which the aforementioned vial remains.

In another aspect, the present application provides a method for diagnosing diseases and/or symptoms associated to expression of an LILRB4 protein in a subject, including: contacting a sample derived from the subject with the isolated antigen-binding protein, and determining the presence and/or amount of a substance capable of specifically binding to the isolated antigen-binding protein in the sample.

In another aspect, the present application provides a method for detecting LILRB4 in a sample or a subject, including administering the isolated antigen-binding protein. In the present application, the administration can be carried out in different ways, such as intravenous, intratumoral, intraperitoneal, subcutaneous, intramuscular, topical or intradermal administration.

Without intending to be limited by any theory, the following examples are only intended to illustrate the antigen-binding proteins, preparation methods and uses of the present application, and are not intended to limit the scope of the invention in the present application.

### Examples

### Example 1 Screening of monoclonal antibody by hybridoma technology

A human LILRB4 protein extracellular domain-human IgG1 Fc fusion Protein (named LILRB4-Fc) was used, in which, the sequence information of the human LILRB4 referred to Q8NHJ6, the sequence of the human IgG1 Fc fragment referred to AXN93652.1, and the amino acid sequence of the human LILRB4 protein extracellular domain was as set forth in SEQ ID NO: 84; and the amino acid sequence of the LILRB4-Fc was as set forth in SEQ ID NO: 85. Balb/c mouse (Beijing Vital River Laboratory Animal Technology Co., Ltd.) was conventionally immunized.

On the days 0, 14 and 28, Balb/c mouse was subcutaneously injected with 100µg of LILRB4-Fc in the presence of a Freund's complete adjuvant (first injection) or Freund's incomplete adjuvant (second and third injections). A conventional hybridoma technical solution (Salhi et al., Biochem.J.2004) was utilized to fuse the mouse splenocyte and mouse myeloma cell SP2/0 (ATCC). Cells were cultured in a plate containing an HAT culture medium (10⁵ cells in each well) for hybridoma selection. After 12 days, supernate was collected, and direct enzyme-linked immunosorbent assay (ELISA) was utilized to screen the clone (positive clone) having binding specificity for LILRB4. The screening was performed to obtain a series of mouse monoclonal antibodies targeting a human LILRB4 extracellular domain (ECD).

The variable region gene of the obtained mouse monoclonal antibody was cloned into an eukaryotic expression vector pCMV-163 containing a human IgG1 constant region gene to construct an antibody expression vector, the antibody expression vector was transfected into CHO-S cells for expression, and a conventional Protein A was used for affining and purifying chimeric antibodies, which were named as 1A2-7, 3A2-18, 3G9-1 and 5E11-2; the amino acid sequences of the chimeric antibodies are disclosed in the specification; and the CDR sequence was subjected to division of a Kabat mode.

| | 1A2-7 | 3A2-18 | 3G9-1 | 5E11-2 |
|---|---|---|---|---|
| HCDR1 | SEQ ID NO: 1 | SEQ ID NO: 1 | SEQ ID NO: 1 | SEQ ID NO: 27 |
| HCDR2 | SEQ ID NO: 2 | SEQ ID NO: 2 | SEQ ID NO: 2 | SEQ ID NO: 28 |
| HCDR3 | SEQ ID NO: 3 | SEQ ID NO: 3 | SEQ ID NO: 20 | SEQ ID NO: 29 |
| VH | SEQ ID NO: 8 | SEQ ID NO: 19 | SEQ ID NO: 22 | SEQ ID NO: 33 |
| LCDR1 | SEQ ID NO: 9 | SEQ ID NO: 9 | SEQ ID NO: 23 | SEQ ID NO: 34 |
| LCDR2 | SEQ ID NO: 10 | SEQ ID NO: 10 | SEQ ID NO: 10 | SEQ ID NO: 35 |
| LCDR3 | SEQ ID NO: 11 | SEQ ID NO: 11 | SEQ ID NO: 24 | SEQ ID NO: 36 |
| VL | SEQ ID NO: 16 | SEQ ID NO: 16 | SEQ ID NO: 26 | SEQ ID NO: 40 |

### Example 2 Binding of chimeric antibodies to human LILRB4

An LILRB4 protein (Human LILRB4/CD85k/ILT3 Protein(ECD, His Tag), Sino Biological) was coated with an ELISA plate strip at 37°C for 2 h; after washing with PBST, 10% of fetal calf serum was added, and sealing was performed at 37°C for 1 h; chimeric antibodies and positive antibody C84 with different concentrations were added, and reacted at 37°C for 1 h; after washing with PBST, Goat anti-human IgG Fc secondary antibody (Goat anti-human IgG Fc antibody, horseradish peroxidase (HRP) conjugate, affinity purified, Invitrogen) marked with horse radish peroxidase was added, and reacted at 37°C for 30 min; washing by PBST was performed for 5 times; 100 µL of TMB (eBioscience) was added to each well, and the mixture was placed in a dark place at room temperature (20±5°C) for 2-3 min; and then 100 µL of 2N H₂SO₄ stop solution was added to each well to terminate the substrate reaction, an OD value was read at 450 nm by an ELIASA, and the binding ability of the chimeric antibodies and LILRB4 was analyzed.

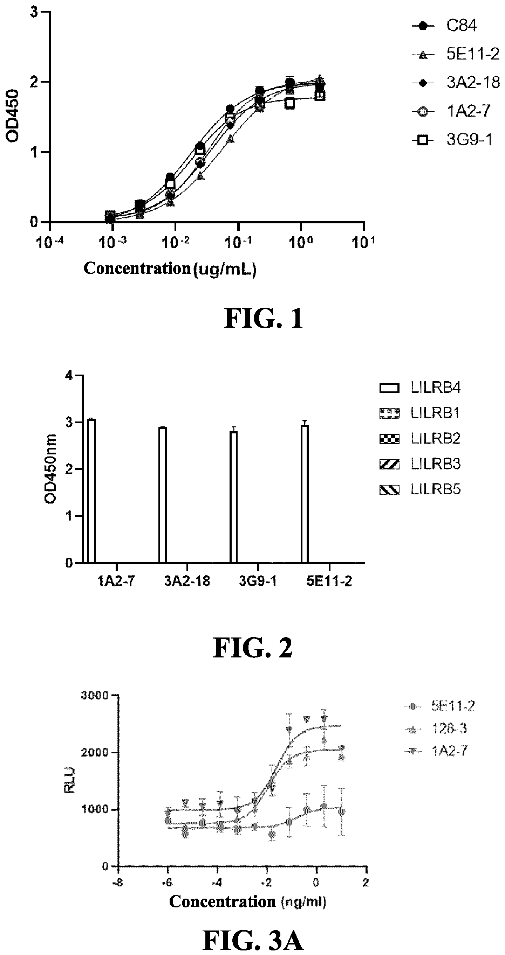

The result is shown in FIG. 1, and the binding ability of the chimeric antibodies 1A2-7, 3A2-18, 3G9-1, 5E11-2 and LILRB4 is similar to that of the positive antibody C84.

### Example 3 Binding specificity detection of chimeric antibodies and human LILRB family

### proteins

LILRB family proteins, namely, LILRB1 (Human LILRB1/CD85/ILT2/ILR1 Protein (ECD, His Tag), Sino Biological), LILRB2 (Human LILRB2/ILT4/LIR-2 Protein (ECD, His Tag), Sino Biological), LILRB3 (Human LILRB3/LIR3/ILT5/CD85a Protein(ECD, His Tag), Sino Biological), LILRB4 (Human LILRB4/CD85k/ILT3 protein (His Tag), Sino Biological), and LILRB5 (Human LILRB5/CD85c/LIR-8 Protein(ECD, His Tag), Sino Biological) proteins were respectively coated with ELISA plate strips and stood overnight at 4°C; after washing with PBST, 10% of fetal calf serum was added, and sealing was carried out at 37°C for 1 h; 10 µg/mL of chimeric antibody was added to each well, and reacted at 37°C for 1 h; after washing with PBST, Goat anti-human IgG Fc secondary antibody (Goat anti-human IgG Fc antibody, horseradish peroxidase (HRP) conjugate, affinity purified, Invitrogen) marked with horse radish peroxidase was added, and reacted at 37°C for 30 min; washing with PBST was carried out for 5 times; 100 µL of TMB (eBioscience) was added to each well, and the mixture was kept in a dark place at room temperature (20±5°C) for 1-2 min; and then 100 µL of 2N H₂SO₄ stop solution was added to each well to terminate the substrate reaction, OD value was read at 450 nm by an ELIASA, and nonspecific binding capacity of the chimeric antibodies with LILRB 1, LILRB2, LILRB3 and LILRB5 was analyzed.

The result is shown in FIG. 2, and the chimeric antibodies do not specifically bind to LILRB 1, LILRB2, LILRB3 and LILRB5.

### Example 4 Detection of activity of chimeric antibodies for blocking LILRB4/APOE binding

The activity of chimeric antibodies for blocking LILRB4/APOE binding was detected by a jurkat-LILRB4-CHO-CD3-APOE reporter gene cell line, and 128-3 (Oncoimmune antibody 128-3) was used as a positive control.

APOE/TCR activator/CHO cells in logarithmic phase were collected, inoculated into a 96-well plate, and cultured at 37°C overnight. On the next day, supernatant was removed, washing with PBS was performed twice, an antibody sample diluted by a 1640 culture medium was added, LILRB4 effector reporter cells in the logarithmic phase were added, and cultured in a 37°C culture box. The cells were taken out after 5 h, bright-Glo luciferase was added, the numerical value was read with an ELIASA, and analyzing was carried out according to the numerical value of each well by prism Graphpad software.

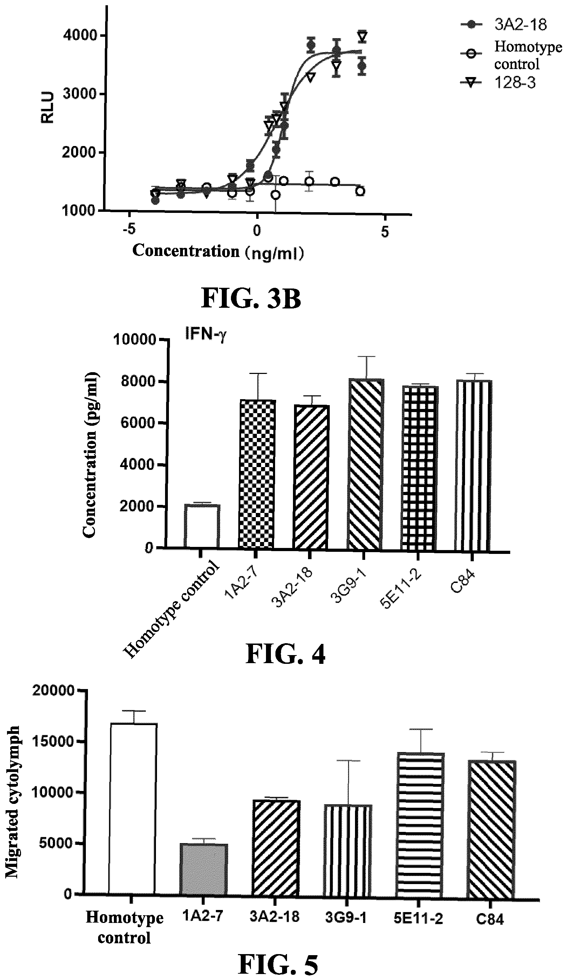

The results are shown in FIG. 3A and FIG. 3B; and the chimeric antibodies 1A2-7 and 3A2-18 can block the binding of LILRB4 and APOE, in which, the blocking activity of 1A2-7 is superior to that of the positive control 128-3.

### Example 5 CD8⁺T cell activation stimulation by chimeric antibodies

PBMC was extracted from whole blood of a healthy supplier, and isolated by a CD8⁺ T cell isolation kit (Milternyi) to obtain CD8⁺ T cells, and the CD8⁺T cells were added to a 96-well plate (96-well Clear Round Bottom TC-treated Microplate, Corning) by 5×10⁴ cells/well; T cell activation magnetic beads (Dynabeads^{®} Human T-Activator CD3/CD28/CD137, GIBCO) were added, and incubated in a 5% CO2 incubator at 37°C for 2 d; and chimeric antibodies with the final concentration of 200 µg/mL, a positive control antibody C84 and a homotype control were added, human mononuclear leukemia cells THP-1 were added according to 5×10⁴ cells/well, and continuously incubated in a 5% CO₂ incubator at 37°C for 5 d, then supernatant was collected, the level of IFN-γ in the supernatant was detected by Human IFN-γ Elisa Kit (ExCell Bio), and the capacity of the chimeric antibodies for activating the CD8⁺T cells in a THP-1/CD8⁺ T lymphocyte co-culture experiment was evaluated.

The result is shown in FIG. 4, and the capacity of the chimeric antibodies 1A2-7, 3A2-18, 3G9-1 and 5E11-2 for activating CD8⁺T cells in THP-1/CD8⁺ T lymphocyte co-culture is similar to that of the positive control antibody C84.

### Example 6 Inhibition of chimeric antibodies on THP-1 cell migration

Human mononuclear leukemia cells THP-1 after starving for 24 h in serum were collected, and added to a 24-well cell culture plate, chimeric antibodies and a positive control antibody C84 with the final concentration of 10 µg/mL were added, and incubated in a 5% CO₂ incubator at 37°C for 1 h; 100 µL of mixed solution of the cells and the antibodies was added to an upper chamber of Transwell (6.5 mm Transwell^{®} with 8.0 µm Pore Polycarbonate Membrane Insert, Corning), 700 µL of complete culture medium was added to a lower chamber, and incubated in a 5% CO2 incubator at 37°C for 24 h; and 100 µL of solution was collected from the lower chamber and added to a 96-well plate (96-well Black/Clear and White/Clear Bottom Polystyrene Microplates, Corning) with a white and transparent bottom, 100 µL of cell activity detection reagent (CellTiter-Lumi^{™} luminescence cell activity detection kit, Beyotime Biotechnology) was added, and incubated in a dark place at room temperature for 10 min, then the relative fluorescence intensity value of chemiluminescence was detected by a multifunctional microplate reader, a standard curve was drawn with gradient diluted THP-1 cells and corresponding relative fluorescence intensity values thereof, the number of cells in the lower chamber was calculated, and the inhibition of the chimeric antibodies on THP-1 cell migration was analyzed.

The result is shown in FIG. 5, the inhibiting capacity of the chimeric antibodies 1A2-7, 3A2-18 and 3G9-1 on the THP-1 cell migration is higher than that of the positive control antibody C84, and the inhibiting capacity of 5E11-2 is similar to that of the positive control antibody C84.

### Example 7 Inhibition of chimeric antibodies on growth and metastasis of THP-1 in NSG mice

PBS resuspended THP1-Luc human monocyte leukemia cells were injected into B-NDG mice in a caudal vein manner by 5×10⁶/ 0.2mL/mouse, the mice were randomly distributed into 5 experimental groups on the day of grouping according to mice imaging signals (average imaging signals: 1.9x10⁷ p/s) and weights (average weights: 22.4 g), and each group included five mice, namely G1(PBS), G2(1A2-7, 10 mg/kg), G3(3A2-18, 10 mg/kg), and G5 (C84, 10 m/kg). Administration was performed on the day of grouping (day 0) and on the day 3 after grouping, and all the mice were subjected to intraperitoneal injection administration. The mice were imaged by a small animal living body imager after cell inoculation (namely before grouping administration, day 0), as well as on days 3, 7, 14, 21, and 25 after administration and before experiment ending (day 26), an imaging signal graph and signal intensity were acquired, the growth condition of tumors in the body was observed, and the experiment was ended after observation was completed. During administration and observation, the animal weights were measured twice a week, and the measured values were recorded.

At the end of an experiment, the tumor imaging signal intensity of a control group (PBS) was 1.43x10⁹±1.32x10⁸ p/s; the tumor imaging signal intensity of a 1A2-7 group was 1.27x10⁶±2.35x10⁵ p/s, and TGITV was 101.3%; the tumor imaging signal intensity of a 3A2-18 group was 1.18x10⁶±2.79x10⁵ p/s, and TGITV was 101.3%; the tumor imaging signal intensity in a C84 group was 3.74x10⁷±1.92x10⁷ p/s, and TGITV was 98.7%. Compared with a G1 group, the P value of each group was less than 0.001, which indicated that the tested products 1A2-7, 3A2-18 and C84 had a significant inhibitory effect on the tumor growth of B-NDG mice transplanted with THP1-LUC cells at a dosage of 10 mg/kg.

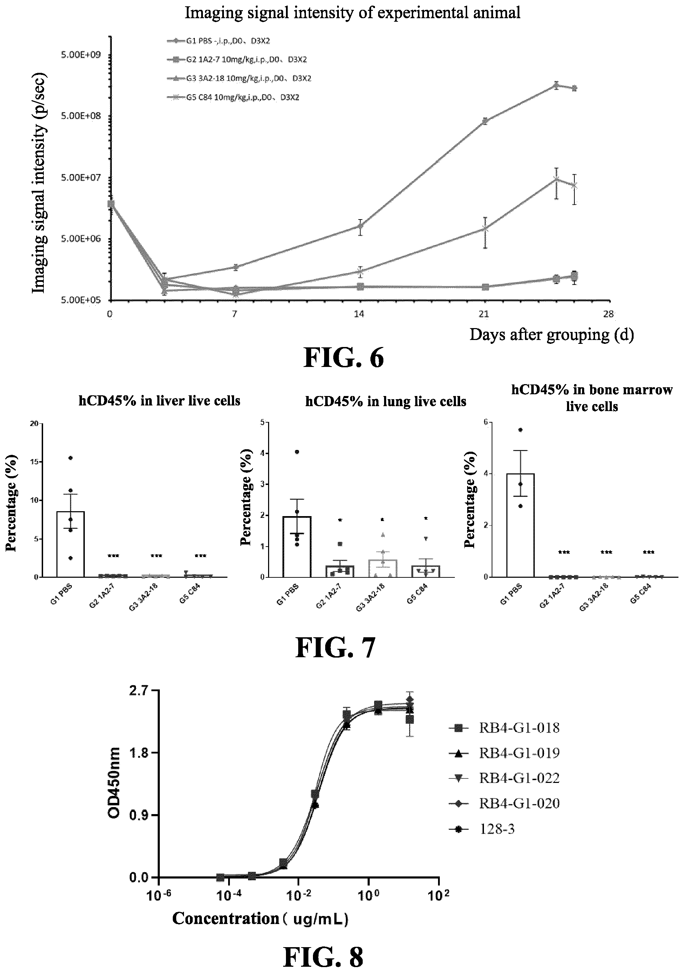

The result is shown in FIG. 6, chimeric antibodies 1A2-7 and 3A2-18 can effectively inhibit the growth of tumors, and the inhibitory effect is significantly better than that of the positive control antibody C84.

At the end of the experiment, after the mice were weighed and imaged, the mice were subjected to euthanasia, and an experimental animal survival curve was drawn; and flow cytometry detection and analysis were performed to analyze the ratio of hCD45⁺ cells (live/dead, hCD45+, and mCD45+) in liver, lung and bone marrow of each group of mice.

Before an experiment of a tumor formation model, flow cytometry detection and analysis indicated that THP1-Luc cells were all hCD45⁺ cells, and there was not mCD45 expression in the cells. Before drug administration, hCD45⁺ cells were detected in the lung, Bone Marrow (BM) and liver of the model mice B-NDG mice constructed in the experiment.

The flow cytometry detection result after drug administration shows that compared with the control group G1, the ratio of hCD45⁺ cells in the liver, lung and bone marrow of each group of mice is significantly reduced, which indicates that the tested products C84, 1A2-7 and 3A2-18 can significantly reduce the amount of tumor cells in the liver, lung and bone marrow of the mice at a dosage of 10 mg/kg, and can inhibit the migration of tumor cells, and the result is shown in FIG. 7.

### Example 8 Humanization of antibodies

Humanized transformation was carried out on antibodies, and a standard CDR grafting method was adopted for humanization. BLAST search was carried out on variable sequences of human heavy chain and light chain, and 3 or 4 sequences were selected from each sequence to serve as humanized receptor frameworks. The heavy chain and light chain CDR1, CDR2 and CDR3 were cloned into 3 different heavy chain frameworks (H1-H4) and 2 different light chain frameworks (L1-L2). Then, the expression level and antigen binding capacity of 4 different antibody combinations (shown in Table 1) in CHO-S cells were detected.

| Name of humanized antibodies | Heavy chain | Light chain |
|---|---|---|
| RB4-G1-018 | SEQ ID NO: 46 | SEQ ID NO: 52 |
| RB4-G1-019 | SEQ ID NO: 56 | SEQ ID NO: 52 |
| RB4-G1-020 | SEQ ID NO: 59 | SEQ ID NO: 52 |
| RB4-G1-022 | SEQ ID NO: 46 | SEQ ID NO: 62 |

### Example 9 Binding of humanized antibody to human LILRB4

An LILRB4 protein (Human LILRB4/CD85k/ILT3 Protein(ECD, His Tag), Sino Biological) was coated with an ELISA plate strip at 37°C for 2 h; after washing with PBST, 10% of fetal calf serum was added, and sealing was performed at 37°C for 1 h; a humanized antibody and a positive antibody 128-3 with different concentrations were added, and reacted at 37°C for 1 h; after washing with PBST, Goat anti-human IgG Fc secondary antibody (Goat anti-human IgG Fc antibody, horseradish peroxidase (HRP) conjugate, affinity purified, Invitrogen) marked with horse radish peroxidase was added, and reacted at 37°C for 30 min; washing by PBST was performed for 5 times; 100 µL of TMB (eBioscience) was added to each well, and the mixture was placed in a dark place at room temperature (20±5°C) for 2-3 min; and then 100 µL of 2N H₂SO₄ stop solution was added to each well to terminate the substrate reaction, an OD value was read at 450 nm by an ELIASA, and the binding ability of the humanized antibody and LILRB4 was analyzed.

The result is shown in FIG. 8, and the binding capacity of the humanized antibodies RB4-G1-018, RB4-G1-019, RB4-G1-020 and RB4-G1-022 to the LILRB4 is similar to that of the positive antibody 128-3.

### Example 10 Affinity determination of humanized antibody

A BIACORE 8K biomacromolecule interactor (GE Company) was adopted to analyze the affinity of an antibody. A Series S Sensor Chip Protein A Chip (GE Company) was used for capturing an antibody to be detected, the concentration of the antibody to be detected was set to be 15 µg/mL, and the sample was fed at a speed of 10 µl/min until RU=500; LILRB4 (Beijing ACROBiosystems, article number: LI4-H52H7, 6×His label, and the theoretical molecular weight: 28.2 Kda) was used as a mobile phase, 6 concentration gradients (0.0977 nM-200 nM) were used, and the binding time was 180 sec; and the dissociation time was 300 sec. The result of the affinity of the antibody to be detected is as set forth in follows:

| **Ligand** | **Analyte** | **ka (1/Ms)** | **kd (1/s)** | **KD (M)** |
|---|---|---|---|---|
| RB4-G1-018 | Human LILRB4 | 1.54E+06 | 4.33E-03 | 2.81E-09 |
| RB4-G1-019 | Human LILRB4 | 1.24E+06 | 3.43E-03 | 2.77E-09 |
| RB4-G1-022 | Human LILRB4 | 1.80E+06 | 4.18E-03 | 2.33E-09 |
| RB4-G1-020 | Human LILRB4 | 2.53E+06 | 8.34E-03 | 3.29E-09 |
| 128-3 | Human LILRB4 | 1.72E+06 | 4.22E-03 | 2.46E-09 |

### Example 11 Determination of binding capacity of humanized antibodies to LILRB4 positive cells

Human mononuclear leukemia cells THP-1 were collected, and added to 1.5 mL EP tubes by 5×10⁵ cells per tube; humanized antibodies and positive control antibodies 128-3 with different concentrations were added, and incubated on ice in a dark place for 30 min; after washing with FACS washing liquid, Goat Anti-Human IgG Fc Secondary Antibody (Invitrogen) marked with PE fluorescence was added, and incubated on ice for 30 min in a dark place; washing by FACS washing liquid was carried out 2 times; 400 µL of 1% paraformaldehyde fixative solution (Solarbio) was added to each tube to fix the cells, and after uniform mixing, relative fluorescence intensity of PE fluorescence was detected on the machine to analyze the binding ability of humanized antibody to LILRB4-positive cells THP-1.

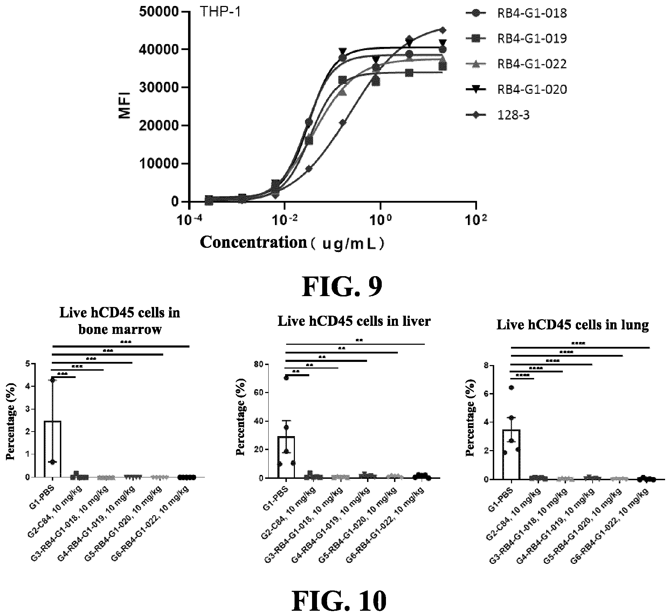

The result is as set forth in FIG. 9, and the activity of the humanized antibodies binding with the LILRB4 positive cells THP-1 is similar and stronger than that of the positive antibody 128-3.

### Example 12 Inhibition of humanized antibody on LILRB4+ tumor migration

PBS resuspended THP1-Luc human monocyte leukemia cells were injected into B-NDG mice in a caudal vein manner by 5×10⁶/0.2mL/mouse. The mice were randomly distributed into 6 experimental groups on the day of inoculation according to mice imaging signals (average imaging signals: 3.6x10⁷p/s) and weights (average weights: 23 g), and each group included 5 mice, namely G1 (PBS), G2 (C84, 10 mg/kg), G3 (RB4-G1-018, 10 mg/kg), G4 (RB4-G1-019, 10 mg/kg), G5 (RB4-G1-020, 10 m/ kg) and G6 (RB4-G1-022, 10 mg/kg). Administration was performed on all the groups of mice by intraperitoneal injection on the day of grouping (day 0) and on the day 3 after grouping. The mice were imaged by a small animal living body imager on the day (day 0) before drug administration by groups, the days 7, 14, 21, and 25 after administration and the day of experiment ending (day 28), an imaging signal graph and signal intensity were acquired, and the growth condition of tumors in the body was observed. During administration and observation, the animal weights were measured twice a week, and the measured values were recorded.

At the end of the experiment, after the mice were weighed and imaged, the mice were subjected to euthanasia, and an experimental animal survival curve was drawn; and flow cytometry detection and analysis were performed to analyze the ratio of hCD45⁺ cells (live/dead, hCD45⁺, and mCD45⁺) in liver, lung and bone marrow of each group of mice.

The flow cytometry detection result is shown in FIG. 10, and compared with the control group G1, the ratio of hCD45⁺ cells in the liver, lung and bone marrow of each group of mice is significantly reduced, which indicates that the tested products C84, RB4-G1-018, RB4-G1-019, RB4-G1-020 and RB4-G1-022 can significantly reduce the amount of tumor cells in the liver, lung and bone marrow of the mice at a dosage of 10 mg/kg.

### Example 13 Detection of activity of LILRB4 humanized antibodies for blocking LILRB4/APOE binding by jurkat-LILRB4-CHO-CD3-APOE reporter cell line

APOE/TCR activator/CHO cells in logarithmic phase were collected, inoculated into a 96-well plate, and cultured at 37°C overnight. On the next day, supernatant was removed, washing with PBS was performed twice, an antibody sample diluted by a 1640 culture medium was added, 50 ul was added to each well, then LILRB4 effector reporter cells in the logarithmic phase were added, and cultured in a 37°C culture box. The cells were taken out after 5 h, bright-Glo luciferase was added, the numerical value was read with an ELIASA, and analyzing was carried out according to the numerical value of each well by prism Graphpad software.

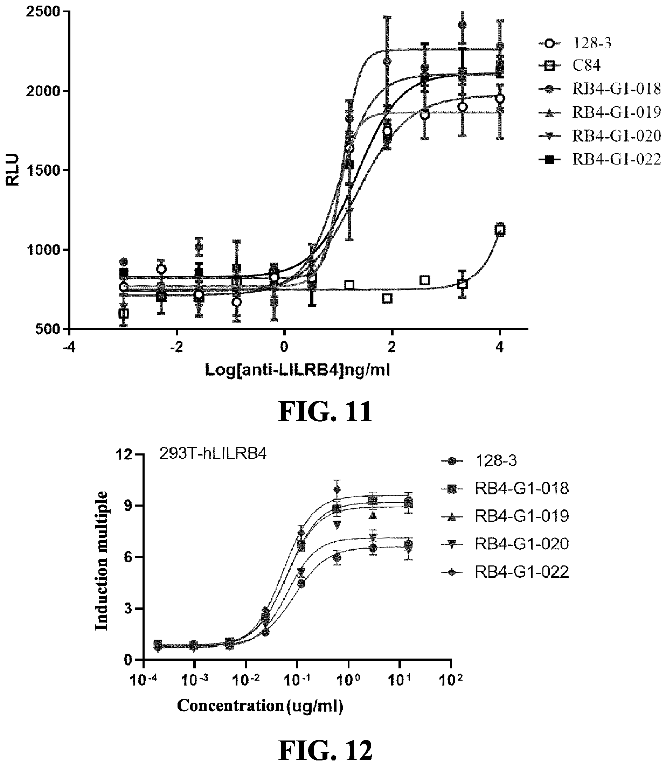

The results are shown in FIG. 11; and the humanized antibodies can block the binding of APOE and LILRB4, and the blocking capacity is superior to that of the positive control antibodies 128-3 and C84.

### Example 14 ADCC activity of humanized antibody on 293T-hLILRB4 cells

293T-hLILRB4 cells overexpressing human LILRB4 gene were collected, and added to a 96-well plate (96-well Black/Clear and White/Clear Bottom Polystyrene Microplates, Corning) with white and transparent bottom; humanized antibodies and positive antibody 128-3 with different concentration were added; effector cells Jurkat-hFcγRIIIa-NFAT overexpressing human FcγRIIIa gene and NFAT fluorescent reporter gene were added, and the 96-well plate was incubated in a 5% CO₂ incubator at 37°C for 6 h; 100 µL of luciferase detection reagent (Bright-Lumi^{™} firefly luciferase reporter gene detection kit, Beyotime Biotechnology) was added to each well, and after 10 min of incubation at room temperature in a dark place, relative fluorescence intensity value of chemiluminescence was detected by a multi-functional ELIASA, and the ADCC activity mediated by humanized antibodies on 293T-hLILRB4 cells was analyzed.

The result is shown in FIG. 12; the ADCC activities of the humanized antibodies RB4-G1-018, RB4-G1-019 and RB4-G1-022 are similar and stronger than those of the positive control antibody 128-3; and the ADCC activity of the humanized antibody RB4-G1-020 is similar to that of the positive control antibody 128-3.

### Example 15 CD8+T cell activation stimulation by humanized antibody

PBMC was extracted from whole blood of a healthy supplier, and isolated by a CD8⁺T cell isolation kit (Milternyi) to obtain CD8⁺T cells, and the CD8+T cells were added to a 96-well plate (96-well Clear Round Bottom TC-treated Microplate, Corning) by 5×10⁴ cells/well; T cell activation magnetic beads (Dynabeads^{®} Human T-Activator CD3/CD28/CD137, GIBCO) were added, and incubated in a 5% CO2 incubator at 37°C for 2 d; and humanized antibodies with the final concentration of 200 µg/mL, a positive antibody 128-3 and a homotype control were added, human mononuclear leukemia cells THP-1 were added according to 5×10⁴ cells/well, and continuously incubated in a 5% CO₂ incubator at 37°C for 5 d, then supernatant was collected, the level of TNF-α in the supernatant was detected by Human TNF-α Elisa Kit (ExCell Bio), and the capacity of the humanized antibodies for activating the CD8⁺T cells in a THP-1/CD8⁺ T lymphocyte co-culture experiment was evaluated.

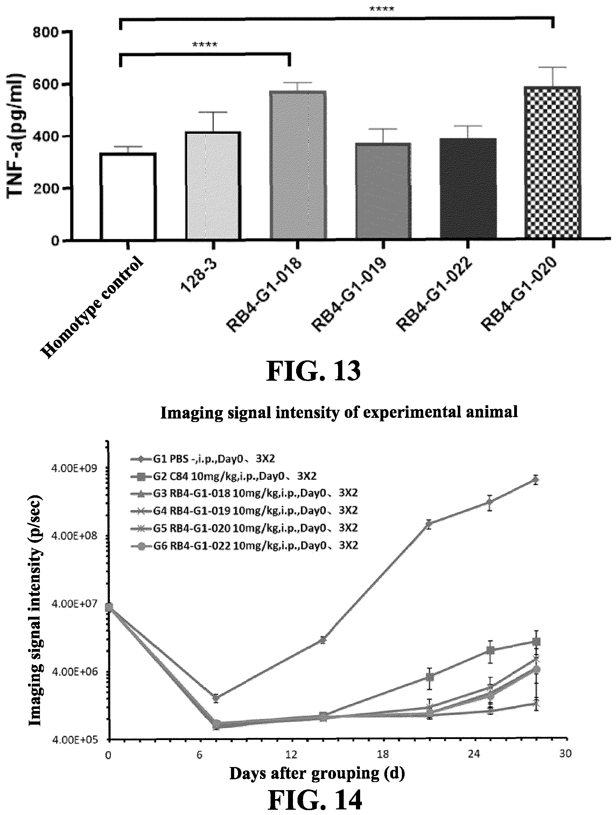

The result is shown in FIG. 13, and the capacity of the humanized antibodies RB4-G1-018 and RB4-G1-020 for activating CD8⁺T cells in THP-1/CD8⁺ T lymphocyte co-culture is stronger than that of the positive antibody 128-3.

### Example 16 Inhibition of LILRB4 humanized antibodies on tumor growth in B-NDG mice with THP1-LUC cell transplantation

PBS resuspended THP1-Luc human monocyte leukemia cells were injected into B-NDG mice in a caudal vein manner by 5×10⁶/0.2mL/mouse. The mice were randomly distributed into 6 experimental groups on the day of inoculation according to mice imaging signals (average imaging signals: 3.6x10⁷ p/s) and weights (average weights: 23 g), and each group included 5 mice, namely G1 (PBS), G2 (C84, 10 mg/kg), G3 (RB4-G1-018, 10 mg/kg), G4 (RB4-G1-019, 10 mg/kg), G5 (RB4-G1-020, 10 m/ kg) and G6 (RB4-G1-022, 10 mg/kg). Administration was performed on all the groups of mice by intraperitoneal injection on the day of grouping (day 0) and on the day 3 after grouping. The mice were imaged by a small animal living body imager on the day (day 0) before drug administration by groups, the days 7, 14, 21, and 25 after administration and the day of experiment ending (day 28), an imaging signal graph and signal intensity were acquired, and the growth condition of tumors in the body was observed. During administration and observation, the animal weights were measured twice a week, and the measured values were recorded.

The result shows that on the day 28 after grouping, the imaging signal intensity of the G1 group of tumors is 2.54x10⁹±3.87x10⁸ p/s; the imaging signal intensity of the G2 group of tumors is 1.06x10⁷±4.50x10⁶ p/s, and the TGITV is 101.0%; the imaging signal intensity of the G3 group of tumors is 4.24x10⁶±1.71x10⁶ p/s, and the TGITV is 101.3%; the imaging signal intensity of the G4 group of tumors is 1.29x10⁶±2.79x10⁵ p/s, and the TGITV is 101.4%; the imaging signal intensity of the G5 group of tumors is 5.82x10⁶±2.35x10⁶ p/s, and the TGITV is 101.2%; and the imaging signal intensity of the G6 group of tumors is 4.08x10⁶±2.63x10⁶ p/s, and the TGITV is 101.3%.

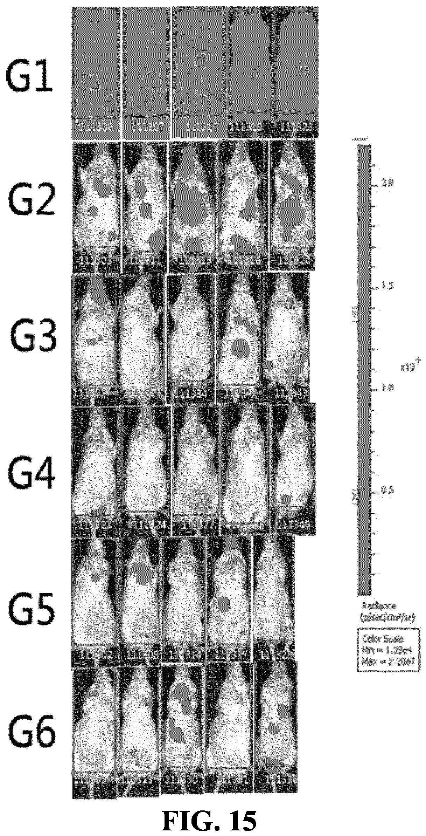

The result is shown in FIG. 14 and FIG. 15, compared with the G1 control group, the P values of G2-G6 are 0.000, which indicates that the samples C84, RB4-G1-018, RB4-G1-019, RB4-G1-020 and RB4-G1-022 have a remarkable inhibition effect on THP1-LUC tumor growth under the dosage of 10 mg/kg. Moreover, the inhibition effect of RB4-G1-018, RB4-G1-019, RB4-G1-020 and RB4-G1-022 is superior to that of C84.

## Claims

1. An isolated antigen-binding protein capable of binding to a leukocyte immunoglobulin-like receptor B4 (LILRB4), comprising at least one CDR in an antibody heavy chain variable region VH, wherein the VH comprises an amino acid sequence as set forth in any one of SEQ ID NO: 81, SEQ ID NO: 33, and SEQ ID NO: 22.

2. The isolated antigen-binding protein according to claim 1, comprising HCDR3, wherein the HCDR3 comprises an amino acid sequence as set forth in SEQ ID NO: 83 or SEQ ID NO: 29.

3. The isolated antigen-binding protein according to claim 2, wherein the HCDR3 comprises an amino acid sequence as set forth in any one of SEQ ID NO: 3, SEQ ID NO: 20 and SEQ ID NO: 29.

4. The isolated antigen-binding protein according to any one of claims 1 to 3, comprising HCDR2, wherein the HCDR2 comprises an amino acid sequence as set forth in SEQ ID NO: 2 or SEQ ID NO: 28.

5. The isolated antigen-binding protein according to any one of claims 1 to 4, comprising HCDR1, wherein the HCDR1 comprises an amino acid sequence as set forth in SEQ ID NO: 69 (X₁X₂WMX₃, X₁ is D or S; X₂ is A or Y; X₃ is D or H).

6. The isolated antigen-binding protein according to claim 5, wherein the HCDR1 comprises an amino acid sequence as set forth in SEQ ID NO: 1 or SEQ ID NO: 27.

7. The isolated antigen-binding protein according to any one of claims 1 to 6, comprising HCDR1, HCDR2 and HCDR3, wherein the HCDR1, HCDR2 and HCDR3 comprise the amino acid sequence selected from the group consisting of:
(1) the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 3;
(2) the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 20; and
(3) the HCDR1 comprises an amino acid sequence as set forth in SEQ ID NO: 27, the HCDR2 comprises an amino acid sequence as set forth in SEQ ID NO: 28, and the HCDR3 comprises an amino acid sequence as set forth in SEQ ID NO: 29.

8. The isolated antigen-binding protein according to any one of claims 1 to 7, comprising H-FR1, wherein a C terminus of the H-FR1 is directly or indirectly connected to an N terminus of the HCDR1, and the H-FR1 comprises an amino acid sequence as set forth in SEQ ID NO: 73.

9. The isolated antigen-binding protein according to claim 8, wherein the H-FR1 comprises an amino acid sequence as set forth in any one of SEQ ID NO: 4, SEQ ID NO: 17, SEQ ID NO: 30 and SEQ ID NO: 41.

10. The isolated antigen-binding protein according to any one of claims 1 to 9, comprising H-FR2, wherein the H-FR2 is located between the HCDR1 and the HCDR2, and the H-FR2 comprises an amino acid sequence as set forth in SEQ ID NO: 74.

11. The isolated antigen-binding protein according to claim 10, wherein the H-FR2 comprises an amino acid sequence as set forth in any one of SEQ ID NO: 5, SEQ ID NO: 31, SEQ ID NO: 42 and SEQ ID NO: 53.

12. The isolated antigen-binding protein according to any one of claims 1 to 11, comprising H-FR3, wherein the H-FR3 is located between the HCDR2 and the HCDR3, and the H-FR3 comprises an amino acid sequence as set forth in SEQ ID NO: 75.

13. The isolated antigen-binding protein according to claim 12, wherein the H-FR3 comprises an amino acid sequence as set forth in any one of SEQ ID NO: 6, SEQ ID NO: 18, SEQ ID NO: 21, SEQ ID NO: 32, SEQ ID NO: 43, SEQ ID NO: 54 and SEQ ID NO: 57.

14. The isolated antigen-binding protein according to any one of claims 1 to 13, comprising H-FR4, wherein an N terminus of the H-FR4 is directly or indirectly connected to a C terminus of the HCDR3, and the H-FR4 comprises an amino acid sequence as set forth in SEQ ID NO: 76.

15. The isolated antigen-binding protein according to claim 14, wherein the H-FR4 comprises an amino acid sequence as set forth in SEQ ID NO: 7 or SEQ ID NO: 44.

16. The isolated antigen-binding protein according to any one of claims 1 to 15, comprising H-FR1, H-FR2, H-FR3 and H-FR4, wherein the H-FR1, H-FR2, H-FR3 and H-FR4 comprise the amino acid sequence selected from the group consisting of:
(1) the H-FR1 comprises an amino acid sequence as set forth in SEQ ID NO: 4, the H-FR2 comprises an amino acid sequence as set forth in SEQ ID NO: 5, the H-FR3 comprises an amino acid sequence as set forth in SEQ ID NO: 6, and the H-FR4 comprises an amino acid sequence as set forth in SEQ ID NO: 7;
(2) the H-FR1 comprises an amino acid sequence as set forth in SEQ ID NO: 17, the H-FR2 comprises an amino acid sequence as set forth in SEQ ID NO: 5, the H-FR3 comprises an amino acid sequence as set forth in SEQ ID NO: 18, and the H-FR4 comprises an amino acid sequence as set forth in SEQ ID NO: 7;
(3) the H-FR1 comprises an amino acid sequence as set forth in SEQ ID NO: 4, the H-FR2 comprises an amino acid sequence as set forth in SEQ ID NO: 5, the H-FR3 comprises an amino acid sequence as set forth in SEQ ID NO: 21, and the H-FR4 comprises an amino acid sequence as set forth in SEQ ID NO: 7;
(4) the H-FR1 comprises an amino acid sequence as set forth in SEQ ID NO: 30, the H-FR2 comprises an amino acid sequence as set forth in SEQ ID NO: 31, the H-FR3 comprises an amino acid sequence as set forth in SEQ ID NO: 32, and the H-FR4 comprises an amino acid sequence as set forth in SEQ ID NO: 7;
(5) the H-FR1 comprises an amino acid sequence as set forth in SEQ ID NO: 41, the H-FR2 comprises an amino acid sequence as set forth in SEQ ID NO: 42, the H-FR3 comprises an amino acid sequence as set forth in SEQ ID NO: 43, and the H-FR4 comprises an amino acid sequence as set forth in SEQ ID NO: 44;
(6) the H-FR1 comprises an amino acid sequence as set forth in SEQ ID NO: 41, the H-FR2 comprises an amino acid sequence as set forth in SEQ ID NO: 53, the H-FR3 comprises an amino acid sequence as set forth in SEQ ID NO: 54, and the H-FR4 comprises an amino acid sequence as set forth in SEQ ID NO: 44;
(7) the H-FR1 comprises an amino acid sequence as set forth in SEQ ID NO: 41, the H-FR2 comprises an amino acid sequence as set forth in SEQ ID NO: 53, the H-FR3 comprises an amino acid sequence as set forth in SEQ ID NO: 57, and the H-FR4 comprises an amino acid sequence as set forth in SEQ ID NO: 44.

17. The isolated antigen-binding protein according to any one of claims 1 to 16, comprising VH, wherein the VH comprises an amino acid sequence as set forth in any one of claims SEQ ID NO: 81, SEQ ID NO: 33 and SEQ ID NO: 22.

18. The isolated antigen-binding protein according to any one of claims 1 to 17, wherein the VH comprises an amino acid sequence as set forth in any one of SEQ ID NO: 8, SEQ ID NO: 19, SEQ ID NO: 22, SEQ ID NO: 33, SEQ ID NO: 45, SEQ ID NO: 55 and SEQ ID NO: 58.

19. The isolated antigen-binding protein according to any one of claims 1 to 18, comprising an antibody heavy chain constant region which is derived from an IgG heavy chain constant region.

20. The isolated antigen-binding protein according to claim 19, wherein the antibody heavy chain constant region is derived from a human IgG heavy chain constant region.

21. The isolated antigen-binding protein according to any one of claims 19 to 20, wherein the antibody heavy chain constant region is derived from a human IgG1 heavy chain constant region or a human IgG4 heavy chain constant region.

22. The isolated antigen-binding protein according to any one of claims 19 to 21, wherein the antibody heavy chain constant region comprises an amino acid sequence as set forth in SEQ ID NO: 63.

23. The isolated antigen-binding protein according to any one of claims 1 to 22, comprising an antibody heavy chain, wherein the antibody heavy chain comprises an amino acid sequence as set forth in any one of SEQ ID NO: 46, SEQ ID NO: 56, SEQ ID NO: 59, SEQ ID NO: 86, SEQ ID NO: 88, SEQ ID NO: 89 and SEQ ID NO: 91.

24. The isolated antigen-binding protein according to any one of claims 1 to 23, comprising at least one CDR in an antibody light chain variable region VL, wherein the VL comprises an amino acid sequence as set forth in SEQ ID NO: 82 or SEQ ID NO: 40.

25. The isolated antigen-binding protein according to any one of claims 1 to 24, comprising LCDR3, wherein the LCDR3 comprises an amino acid sequence as set forth in SEQ ID NO: 72 (QX₁X₂X₃X₄X₅PX₆T, X₁ is H or Q; X₂ is G or S; X₃ is D, N or W; X₄ is E or T; X₅ is I or L; and X₆ is P or R).

26. The isolated antigen-binding protein according to claim 25, wherein the LCDR3 comprises an amino acid sequence as set forth in any one of SEQ ID NO: 11, SEQ ID NO: 24 and SEQ ID NO: 36.

27. The isolated antigen-binding protein according to any one of claims 1 to 26, comprising LCDR2, wherein the LCDR2 comprises an amino acid sequence as set forth in SEQ ID NO: 71.

28. The isolated antigen-binding protein according to claim 27, wherein the LCDR2 comprises an amino acid sequence as set forth in SEQ ID NO: 10 or SEQ ID NO: 35.

29. The isolated antigen-binding protein according to any one of claims 1 to 28, comprising LCDR1, wherein the LCDR1 comprises an amino acid sequence as set forth in SEQ ID NO: 70 or SEQ ID NO: 34.

30. The isolated antigen-binding protein according to claim 29, wherein the LCDR1 comprises an amino acid sequence as set forth in any one of SEQ ID NO: 9, SEQ ID NO: 23 and SEQ ID NO: 34.

31. The isolated antigen-binding protein according to any one of claims 1 to 30, comprising LCDR1, LCDR2 and LCDR3, wherein the LCDR1, LCDR2 and LCDR3 comprise the amino acid sequence selected from the group consisting of:
(1) the LCDR1 comprises an amino acid sequence as set forth in SEQ ID NO: 9, the LCDR2 comprises an amino acid sequence as set forth in SEQ ID NO: 10, and the LCDR3 comprises an amino acid sequence as set forth in SEQ ID NO: 11;
(2) the LCDR1 comprises an amino acid sequence as set forth in SEQ ID NO: 23, the LCDR2 comprises an amino acid sequence as set forth in SEQ ID NO: 10, and the LCDR3 comprises an amino acid sequence as set forth in SEQ ID NO: 24; and
(3) the LCDR1 comprises an amino acid sequence as set forth in SEQ ID NO: 34, the LCDR2 comprises an amino acid sequence as set forth in SEQ ID NO: 35, and the LCDR3 comprises an amino acid sequence as set forth in SEQ ID NO: 36.

32. The isolated antigen-binding protein according to any one of claims 1 to 31, comprising L-FR1, wherein a C terminus of the L-FR1 is directly or indirectly connected to an N terminus of the LCDR1, and the L-FR1 comprises an amino acid sequence as set forth in SEQ ID NO: 77.

33. The isolated antigen-binding protein according to claim 32, wherein the L-FR1 comprises an amino acid sequence as set forth in any one of SEQ ID NO: 12, SEQ ID NO: 25, SEQ ID NO: 37 and SEQ ID NO: 47.

34. The isolated antigen-binding protein according to any one of claims 1 to 33, comprising L-FR2, wherein the L-FR2 is located between the LCDR1 and the LCDR2, and the L-FR2 comprises an amino acid sequence as set forth in SEQ ID NO: 78.

35. The isolated antigen-binding protein according to claim 34, wherein the L-FR2 comprises an amino acid sequence as set forth in any one of SEQ ID NO: 13, SEQ ID NO: 38 and SEQ ID NO: 48.

36. The isolated antigen-binding protein according to any one of claims 1 to 35, comprising L-FR3, wherein the L-FR3 is located between the LCDR2 and the LCDR3, and the L-FR3 comprises an amino acid sequence as set forth in SEQ ID NO: 79.

37. The isolated antigen-binding protein according to claim 36, wherein the L-FR3 comprises an amino acid sequence as set forth in any one of SEQ ID NO: 14, SEQ ID NO: 39, SEQ ID NO: 49 and SEQ ID NO: 60.

38. The isolated antigen-binding protein according to any one of claims 1 to 37, comprising L-FR4, wherein an N terminus of the L-FR4 is directly or indirectly connected to a C terminus of the LCDR3, and the L-FR4 comprises an amino acid sequence as set forth in SEQ ID NO: 80.

39. The isolated antigen-binding protein according to claim 38, wherein the L-FR4 comprises an amino acid sequence as set forth in SEQ ID NO: 15 or SEQ ID NO: 50.

40. The isolated antigen-binding protein according to any one of claims 1 to 39, comprising L-FR1, L-FR2, L-FR3 and L-FR4, wherein the L-FR1, L-FR2, L-FR3 and L-FR4 comprise the amino acid sequence selected from the group consisting of:
(1) the L-FR1 comprises an amino acid sequence as set forth in SEQ ID NO: 12, the L-FR2 comprises an amino acid sequence as set forth in SEQ ID NO: 13, the L-FR3 comprises an amino acid sequence as set forth in SEQ ID NO: 14, and the L-FR4 comprises an amino acid sequence as set forth in SEQ ID NO: 15;
(2) the L-FR1 comprises an amino acid sequence as set forth in SEQ ID NO: 25, the L-FR2 comprises an amino acid sequence as set forth in SEQ ID NO: 13, the L-FR3 comprises an amino acid sequence as set forth in SEQ ID NO: 14, and the L-FR4 comprises an amino acid sequence as set forth in SEQ ID NO: 15;
(3) the L-FR1 comprises an amino acid sequence as set forth in SEQ ID NO: 37, the L-FR2 comprises an amino acid sequence as set forth in SEQ ID NO: 38, the L-FR3 comprises an amino acid sequence as set forth in SEQ ID NO: 39, and the L-FR4 comprises an amino acid sequence as set forth in SEQ ID NO: 15;
(4) the L-FR1 comprises an amino acid sequence as set forth in SEQ ID NO: 47, the L-FR2 comprises an amino acid sequence as set forth in SEQ ID NO: 48, the L-FR3 comprises an amino acid sequence as set forth in SEQ ID NO: 49, and the L-FR4 comprises an amino acid sequence as set forth in SEQ ID NO: 50; and
(5) the L-FR1 comprises an amino acid sequence as set forth in SEQ ID NO: 47, the L-FR2 comprises an amino acid sequence as set forth in SEQ ID NO: 48, the L-FR3 comprises an amino acid sequence as set forth in SEQ ID NO: 60, and the L-FR4 comprises an amino acid sequence as set forth in SEQ ID NO: 50.

41. The isolated antigen-binding protein according to any one of claims 1 to 40, comprising VL, wherein the VL comprises an amino acid sequence as set forth in SEQ ID NO: 82 or SEQ ID NO: 40.

42. The isolated antigen-binding protein according to claims 24 to 41, wherein the VL comprises an amino acid sequence as set forth in any one of SEQ ID NO: 16, SEQ ID NO: 26, SEQ ID NO: 40, SEQ ID NO: 51 and SEQ ID NO: 61.

43. The isolated antigen-binding protein according to any one of claims 1 to 42, comprising an antibody light chain constant region which comprises a human Igκ constant region.

44. The isolated antigen-binding protein according to claim 43, wherein the antibody light chain constant region comprises an amino acid sequence as set forth in any one of SEQ ID NO: 64.

45. The isolated antigen-binding protein according to any one of claims 43 to 44, comprising an antibody light chain, wherein the antibody light chain comprises an amino acid sequence as set forth in any one of SEQ ID NO: 52, SEQ ID NO: 62, SEQ ID NO: 87, SEQ ID NO: 90 and SEQ ID NO: 92.

46. The isolated antigen-binding protein according to any one of claims 1 to 45, comprising HCDR1, HCDR2, HCDR3, LCDR1, LCDR2 and LCDR3, wherein the HCDR1, HCDR2, HCDR3, LCDR1, LCDR2 and LCDR3 comprise the amino acid sequence selected from the group consisting of:
(1) the HCDR1 comprises an amino acid sequence as set forth in SEQ ID NO: 1, the HCDR2 comprises an amino acid sequence as set forth in SEQ ID NO: 2, the HCDR3 comprises an amino acid sequence as set forth in SEQ ID NO: 3, the LCDR1 comprises an amino acid sequence as set forth in SEQ ID NO: 9, the LCDR2 comprises an amino acid sequence as set forth in SEQ ID NO: 10, and the LCDR3 comprises an amino acid sequence as set forth in SEQ ID NO: 11;
(2) the HCDR1 comprises an amino acid sequence as set forth in SEQ ID NO: 1, the HCDR2 comprises an amino acid sequence as set forth in SEQ ID NO: 2, the HCDR3 comprises an amino acid sequence as set forth in SEQ ID NO: 20, the LCDR1 comprises an amino acid sequence as set forth in SEQ ID NO: 23, the LCDR2 comprises an amino acid sequence as set forth in SEQ ID NO: 10, and the LCDR3 comprises an amino acid sequence as set forth in SEQ ID NO: 24; and
(3) the HCDR1 comprises an amino acid sequence as set forth in SEQ ID NO: 27, the HCDR2 comprises an amino acid sequence as set forth in SEQ ID NO: 28, the HCDR3 comprises an amino acid sequence as set forth in SEQ ID NO: 29, the LCDR1 comprises an amino acid sequence as set forth in SEQ ID NO: 34, the LCDR2 comprises an amino acid sequence as set forth in SEQ ID NO: 35, and the LCDR3 comprises an amino acid sequence as set forth in SEQ ID NO: 36.

47. The isolated antigen-binding protein according to any one of claims 1 to 46, comprising VH and VL, wherein the VH and VL comprise the amino acid sequence selected from the group consisting of:
(1) the VH comprises an amino acid sequence as set forth in SEQ ID NO: 8, and the VL comprises an amino acid sequence as set forth in SEQ ID NO: 16;
(2) the VH comprises an amino acid sequence as set forth in SEQ ID NO: 19, and the VL comprises an amino acid sequence as set forth in SEQ ID NO: 16;
(3) the VH comprises an amino acid sequence as set forth in SEQ ID NO: 22, and the VL comprises an amino acid sequence as set forth in SEQ ID NO: 26;
(4) the VH comprises an amino acid sequence as set forth in SEQ ID NO: 33, and the VL comprises an amino acid sequence as set forth in SEQ ID NO: 40;
(5) the VH comprises an amino acid sequence as set forth in SEQ ID NO: 45, and the VL comprises an amino acid sequence as set forth in SEQ ID NO: 51;
(6) the VH comprises an amino acid sequence as set forth in SEQ ID NO: 55, and the VL comprises an amino acid sequence as set forth in SEQ ID NO: 51;
(7) the VH comprises an amino acid sequence as set forth in SEQ ID NO: 58, and the VL comprises an amino acid sequence as set forth in SEQ ID NO: 51; and
(8) the VH comprises an amino acid sequence as set forth in SEQ ID NO: 45, and the VL comprises an amino acid sequence as set forth in SEQ ID NO: 61.

48. The isolated antigen-binding protein according to any one of claims 1 to 47, comprising the antibody heavy chain and the antibody light chain, wherein the antibody heavy chain and the antibody light chain comprise the amino acid sequence selected from the group consisting of:
(1) the antibody heavy chain comprises an amino acid sequence as set forth in SEQ ID NO: 86, and the antibody light chain comprises an amino acid sequence as set forth in SEQ ID NO: 87;
(2) the antibody heavy chain comprises an amino acid sequence as set forth in SEQ ID NO: 88, and the antibody light chain comprises an amino acid sequence as set forth in SEQ ID NO: 87;
(3) the antibody heavy chain comprises an amino acid sequence as set forth in SEQ ID NO: 89, and the antibody light chain comprises an amino acid sequence as set forth in SEQ ID NO: 90;
(4) the antibody heavy chain comprises an amino acid sequence as set forth in SEQ ID NO: 91, and the antibody light chain comprises an amino acid sequence as set forth in SEQ ID NO: 92;
(5) the antibody heavy chain comprises an amino acid sequence as set forth in SEQ ID NO: 46, and the antibody light chain comprises an amino acid sequence as set forth in SEQ ID NO: 52;
(6) the antibody heavy chain comprises an amino acid sequence as set forth in SEQ ID NO: 56, and the antibody light chain comprises an amino acid sequence as set forth in SEQ ID NO: 52;
(7) the antibody heavy chain comprises an amino acid sequence as set forth in SEQ ID NO: 59, and the antibody light chain comprises an amino acid sequence as set forth in SEQ ID NO: 52; and
(8) the antibody heavy chain comprises an amino acid sequence as set forth in SEQ ID NO: 46, and the antibody light chain comprises an amino acid sequence as set forth in SEQ ID NO: 62.

49. The isolated antigen-binding protein according to any one of claims 1 to 48, comprising an antibody or antigen-binding fragment thereof.

50. The isolated antigen-binding protein according to claim 49, wherein the antibody is selected from the group consisting of a monoclonal antibody, a single-chain antibody, a chimeric antibody, a multispecific antibody, a humanized antibody and a fully human antibody.

51. The isolated antigen-binding protein according to any one of claims 49 to 50, wherein the antigen-binding fragment is selected from the group consisting of Fab, Fab', F(ab)2, Fv, F(ab')2, scFv, di-scFv and dAb fragments.

52. The isolated antigen-binding protein according to any one of claims 1 to 51, comprising one or more of the following properties:
1) specifically recognizing the leukocyte immunoglobulin-like receptor B4 (LILRB4);
2) binding to LILRB4 protein at a KD value of 2x10-7M or less;
3) blocking the binding activity of LILRB4/APOE;
4) stimulating the activation of CD8+T cell;
5) inhibiting the migration of human mononuclear leukemia cell THP-1;
6) inhibiting the growth and/or metastasis of tumor cells; and
7) blocking the binding of LILRB4 to Fibronectin.

53. The isolated antigen-binding protein according to claim 52, wherein the LILRB4 is human LILRB4.

54. Polypeptide, comprising the isolated antigen-binding protein according to any one of claims 1 to 53.

55. A fusion protein, comprising the isolated antigen-binding protein according to any one of claims 1 to 53.

56. A drug molecule, comprising the isolated antigen-binding protein according to any one of claims 1 to 53.

57. One or a plurality of isolated nucleic acid molecules, encoding the isolated antigen-binding protein according to any one of claims 1 to 53.

58. A vector, comprising the nucleic acid molecules according to claim 57.

59. A cell, comprising the nucleic acid molecules according to claim 57 or the vector according to claim 58.

60. A pharmaceutical composition, comprising the isolated antigen-binding protein according to any one of claims 1 to 53, the polypeptide according to claim 54, the fusion protein according to claim 55, the drug molecule according to claim 56, the nucleic acid molecules according to claim 57, the vector according to claim 58 and/or the cell according to claim 59, and optionally a pharmaceutically acceptable carrier.

61. The pharmaceutical composition according to claim 60, further comprising one or more drugs selected from the following group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

62. A method for preparing the isolated antigen-binding protein according to any one of claims 1 to 53, comprising: culturing the cell according to claim 59 by expressing with the isolated antigen-binding protein according to any one of claims 1 to 53.

63. Use of the isolated antigen-binding protein according to any one of claims 1 to 53, the polypeptide according to claim 54, the fusion protein according to claim 55, the drug molecule according to claim 56, the nucleic acid molecules according to claim 57, the vector according to claim 58, the cell according to claim 59, and/or the pharmaceutical composition according to any one of claims 60 to 61 in preparation of a drug which is used for preventing and/or treating diseases and/or symptoms, wherein the diseases and/or symptoms comprise diseases and/or symptoms associated to LILRB4 signaling.

64. The use according to claim 63, wherein the diseases and/or symptoms comprise tumor.

65. The use according to claim 64, wherein the tumor comprises a solid tumor and/or a blood tumor.

66. The use according to any one of claims 63 to 65, wherein the drug is used in combination with one or more of the drugs selected from the group consisting of: an anthracycline topoisomerase inhibitor, daunorubicin, a cytarabine nucleoside metabolism inhibitor, combined daunorubicin, cytarabine, daunorubicin and cytarabine liposome injection, Vyxeos, all-trans retinoic acid (ATRA), arsenic, arsenic trioxide, histamine hydrochloride, Ceplene, interleukin-2, interleukin-2 (Proleukin), Mylotarg, clofarabine, a farnesyl transferase inhibitor, decitabine, an IDH1 inhibitor, an IDH2 inhibitor, entedipine, Idhifa, an IDO inhibitor, epacadostat, a derivative of platinum complexes, oxaliplatin, a kinase inhibitor, a tyrosine kinase inhibitor, a PI3 kinase inhibitor, a BTK inhibitor Ibrutinib, a PD-1 antibody, a PD-L1 antibody, an anti-CTLA-4 antibody, an LAG3 antibody, an ICOS antibody, a TIGIT antibody, a TIM3 antibody, a tumor antigen-binding antibody, a T cell surface marker binding antibody, a cell or NK cell surface marker binding antibody, an alkylating agent, a nitrosourea agent, antimetabolites, antitumor antibiotics, plant-derived alkaloids, a topoisomerase inhibitor, hormonal therapy medications, a hormone antagonist, an aromatase inhibitor, and a P-glycoprotein inhibitor.

67. The use according to any one of claims 63 to 66, wherein the diseases and/or symptoms associated to LILRB4 signaling comprise immune system diseases and/or malignant blood diseases.

68. The use according to claim 67, wherein the immune system disease is selected from one or more of the group consisting of kawasaki disease, Systemic Lupus Erythematosus (SLE), and sepsis.

69. The use according to any one of claims 65 to 68, wherein the solid tumor is selected from one or more of the group consisting of breast cancer, melanoma, colon cancer, lung cancer, kidney cancer, and pancreatic cancer.

70. The use according to any one of claims 67 to 69, wherein the malignant blood disease is selected from one or more of the group consisting of myelodysplastic syndrome, myeloproliferative tumor, chronic myelomonocytic leukemia (CMML), chronic myelocytic leukemia, or Acute Myelocytic Leukemia (AML), Acute Promyelocytic Leukemia (APL) or M3 AML, acute myelomonocytic leukemia or M4 AML, acute monocytic leukemia or M5 AML, acute myeloblastic leukemia, and polycythemia vera.

71. A method for diagnosing a disease or symptom associated with expression of LILRB4 protein in a subject, comprising: contacting a sample derived from a subject with the isolated antigen-binding protein according to any one of claims 1 to 53, and determining the presence and/or amount of a substance capable of specifically binding to the isolated antigen-binding protein in the sample.

72. A method for detecting LILRB4 protein in a sample, comprising: administering the isolated antigen-binding protein according to any one of claims 1 to 53.

73. The detection method according to claim 72, which is an in vitro and/or ex vivo method.

74. A detection kit, comprising the isolated antigen-binding protein according to any one of claims 1 to 53, wherein the detection kit is used for detecting the presence and/or amount of LILRB4 protein in a sample or subject.
